(19) Europäisches Patentamt
European Patent Office
Office européen des brevets

(11) EP 4 656 636 A1

(12) EUROPEAN PATENT APPLICATION
published in accordance with Art. 153(4) EPC

(43) Date of publication:
**03.12.2025 Bulletin 2025/49**

(21) Application number: **24746993.5**

(22) Date of filing: **26.01.2024**

(51) International Patent Classification (IPC):
***C07D 401/12*** (2006.01) ***C07D 401/14*** (2006.01)
***C07D 403/14*** (2006.01) ***A61K 31/4412*** (2006.01)
***A61K 31/444*** (2006.01) ***A61P 35/00*** (2006.01)

(52) Cooperative Patent Classification (CPC):
**A61K 31/4412; A61K 31/444; A61P 35/00; C07D 401/12; C07D 401/14; C07D 403/14**

(86) International application number:
**PCT/CN2024/074207**

(87) International publication number:
**WO 2024/156286 (02.08.2024 Gazette 2024/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.01.2023 CN 202310072130**
**28.01.2023 CN 202310072098**

(71) Applicant: **QILU PHARMACEUTICAL CO., LTD.**
**Jinan, Shandong 250100 (CN)**

(72) Inventors:
- **FU, Qiang**
**Jinan, Shandong 250100 (CN)**
- **JIANG, Chen**
**Jinan, Shandong 250100 (CN)**

(74) Representative: **Altmann Stößel Dick Patentanwälte PartG mbB**
**Theodor-Heuss-Anlage 2**
**68165 Mannheim (DE)**

# (54) CRYSTAL FORM AND USE OF PARP 7 INHIBITOR AND SALT THEREOF

(57) A crystal form of a compound of formula (I) that serves as a PARP7 inhibitor, a crystal form of a pharmaceutically acceptable salt of the compound of formula (I), and the use thereof in the preparation of a drug for treating related diseases. (FIG. 1)

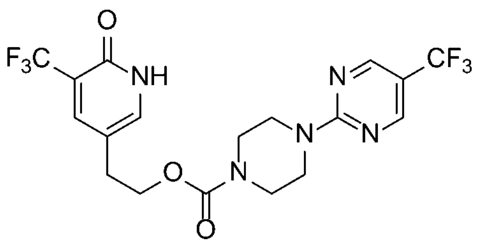

(I).

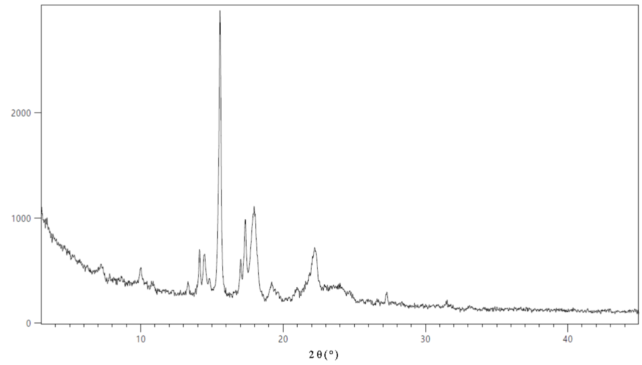


FIG. 1

## Description

The present application claims the priorities to Chinese Patent Application No. CN202310072130.5, filed before the China National Intellectual Property Administration (CNIPA) on January 28, 2023, titled "CRYSTAL FORM OF PARP7 INHIBITOR AND PREPARATION METHOD THEREOF", and Chinese Patent Application No. CN202310072098.0, filed before the China National Intellectual Property Administration (CNIPA) on January 28, 2023, titled "SALT AND CRYSTAL FORM OF PARP7 INHIBITOR AND PREPARATION METHOD THEREOF", which are incorporated herein by reference in their entirety.

## Technical Field

The present application relates to the field of medicinal chemistry, in particular to crystal form and use of PARP7 inhibitor and salt thereof.

## Background

The poly(ADP-ribose) polymerase (PARP) family consists of 17 members that regulate fundamental cellular processes including gene expression, protein degradation, and multicellular stress responses (CoHen, P. Chang, Insights into the biogenesis, function, and regulation of ADP-ribosylation. Nat Chem Biol 14, 236-243(2018)). The ability of cancer cells to survive stress is a fundamental cancer mechanism and a new avenue for novel therapeutic agents.

The 17 members of the PARP family are identified in the human genome based on homology within the catalytic domain. However, based on different activities, they are divided into three subfamilies, polyPARPs, monoPARPs, and inactive (S.Vyas et al. Family-wide analysis of poly (ADP-ribose) polymerase activity. Nat Commun 5, 4426(2014)). Most PARP family members catalyze the transfer of mono(ADP-ribose) units on their substrates (MonoPARPs), other members (PARP1, PARP2, TNKS, TNKS2) catalyze the transfer of poly(ADP-ribose) units on substrates (PolyPARPs), while PARP13 is the only PARP so far that does not show catalytic activity in vitro or in vivo. The above mentioned MonoPARPs modify the target through a single ADP-ribose unit to achieve regulation of signal transduction pathways, a process similar to kinase phosphorylation. The PolyPARPs described above modify their protein targets through large branched polymers called poly(ADP-ribose), a large and highly charged attachment that forms a protein scaffold that guides the protein to function at specific sites within the cell.

PARP1 of the PARP family has been shown to be a potent cancer target associated with DNA damage-induced cellular stress induced by genetic mutations or cytotoxic chemotherapy. There are four clinically approved drugs, as well as several others in late-stage development (A. Ohmoto, S. Yachida, Current status of poly(ADP-ribose) polymerase inhibitors and future directions. Onco Targets Ther 10, 5195-5208(2017)).

The PARP7 gene is located on chromosome 3 (3q25), which is in a region of frequent amplification in squamous histologic cancers. Genome-wide association studies have identified susceptibility genes for ovarian cancer, suggesting a role for PARP7 in this type of cancer (E. L. Goode et al. A genome-wide association study identifies susceptibility loci for ovarian cancer at 2q31 and 8q24. Nat Genet 42, 874-879(2010)). PARP7 has multiple cellular functions. Under AHR signaling, PARP7 acts as a negative feedback mechanism to regulate the expression of P4501A1 and P4501B1. PARP7 has also been described to affect hepatic X receptor ADP-ribonucleic acid, thereby regulating its transcriptional activity (C.Bigetsboll et al. TCDD-Inducible poly-ADP-ribose (TIPARP/PARP7) mono-ADP-ribosylates and co-activates liver X receptors. Biochem J 473, 899-910(2016)). During viral infection, PARP7 can bind to Sindbis virus to promote viral RNA degradation. Furthermore, in the context of viral infection, AHR-induced PARP7 can interact with TBK1, a major kinase during the onset of pathogen-associated molecular pattern pathways, thereby activating the type I interferon response and antiviral immunity. PARP7 has been shown to ADP-ribosylate TBK1, preventing TBK1 activation and thereby inhibiting the type I interferon response.

Studies have shown that many cancer cells rely on PARP7 for intrinsic cell survival while PARP7 enables cancer cells to "hide" outside the immune system. Therefore, inhibition of PARP7 can effectively inhibit cancer cell growth, restore interferon signaling, and effectively suppress cancer. PARP7 inhibitors have demonstrated sustained tumor growth inhibition, potent anti-proliferative activity, and restoration of interferon signaling in several cancer models. In vivo models also demonstrated induction of tumor-specific adaptive immune memory. This effect is mediated by activating IFN-β signaling in immune cells and developing immune memory to enhance immune system signaling and achieve immune memory for tumor suppression. Studies have shown favorable in vivo efficacy of PARP7 alone. Compounds of this target have been found to effectively inhibit PARP7 enzymatic activity. Therefore, by further development of crystalline forms and preparation methods of this class of compounds, as well as pharmaceutical compositions in their crystalline forms, may provide more effective treatment for tumor patients.

## Summary of the invention

The present application provides a crystalline form of a compound of formula (I), a salt of the compound of formula (I) and a crystalline form thereof, and use thereof.

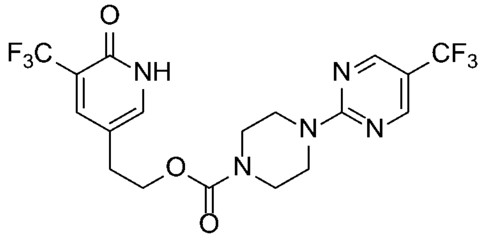

(I).

In a first aspect of the present application, a crystal form IV of a compound of formula (I) is provided, which has characteristic peak(s) at 1, 2, 3, 4, or 5 of 2θ values of 14.13°, 15.56°, 17.33°, 18.07°, and 22.30°, each with a 2θ error range of ± 0.2° in an X-ray powder diffraction pattern as measured using Cu-$K_\alpha$ radiation.

In some embodiments of the present application, the X-ray powder diffraction pattern of the crystal form IV has characteristic peaks at 2θ values of 14.13°, 15.56°, 17.33°, 18.07°, and 22.30°, each with a 2θ error range of ± 0.2°, as measured using Cu-$K_\alpha$ radiation.

In some embodiments of the present application, the X-ray powder diffraction pattern of the crystal form IV has characteristic peak(s) at 1, 2, 3, 4, 5, 6, 7, or 8 of 2θ values of 14.13°, 14.46°, 15.56°, 17.02°, 17.33°, 18.07°, 19.17°, and 22.30°, each with a 2θ error range of ± 0.2°, as measured using Cu-$K_\alpha$ radiation.

In some embodiments of the present application, the X-ray powder diffraction pattern of the crystal form IV has characteristic peaks at 2θ values of 14.13°, 14.46°, 15.56°, 17.02°, 17.33°, 18.07°, 19.17°, and 22.30°, each with a 2θ error range of ± 0.2°, as measured using Cu-$K_\alpha$ radiation.

In some embodiments of the present application, the X-ray powder diffraction pattern of the crystal form IV has characteristic peak(s) at 1, 2, 3, 4, 5, 6, 7, 8, 9, or 10 of 2θ values of 7.25°, 10.00°, 14.13°, 14.46°, 15.56°, 17.02°, 17.33°, 18.07°, 19.17°, and 22.30°, each with a 2θ error range of ± 0.2°, as measured using Cu-$K_\alpha$ radiation.

In some embodiments of the present application, the X-ray powder diffraction pattern of the crystal form IV has characteristic peaks at 2θ values of 7.25°, 10.00°, 14.13°, 14.46°, 15.56°, 17.02°, 17.33°, 18.07°, 19.17°, and 22.30°, each with a 2θ error range of ± 0.2°, as measured using Cu-$K_\alpha$ radiation.

In some embodiments of the present application, the X-ray powder diffraction pattern of the crystal form IV is substantially as shown in FIG. 1.

In some embodiments of the present application, the X-ray powder diffraction peak analytical data of the crystal form IV is shown in Table 1.

Table 1 Analytical data of XRPD diffraction peaks of the crystal form IV of the compound of formula (I)

| No. | 2Theta (°) | Intensity (counts) | Interplanar spacing (Å) | Relative intensity (%) |
|---|---|---|---|---|
| 1 | 7.25 | 116.18 | 12.18996 | 4.27 |
| 2 | 10.00 | 184.53 | 8.8454 | 6.79 |
| 3 | 10.83 | 53.96 | 8.1704 | 1.98 |
| 4 | 13.34 | 107.02 | 6.63569 | 3.94 |
| 5 | 14.13 | 438.24 | 6.26813 | 16.11 |
| 6 | 14.46 | 383.85 | 6.12389 | 14.11 |
| 7 | 15.56 | 2719.54 | 5.69423 | 100 |
| 8 | 17.02 | 373.86 | 5.2085 | 13.75 |
| 9 | 17.33 | 754 | 5.1177 | 27.73 |
| 10 | 18.07 | 763.29 | 4.9097 | 28.07 |
| 11 | 19.17 | 152.34 | 4.63073 | 5.6 |
| 12 | 20.97 | 107.61 | 4.2366 | 3.96 |

(continued)

| No. | 2Theta (°) | Intensity (counts) | Interplanar spacing (Å) | Relative intensity (%) |
|---|---|---|---|---|
| 13 | 22.30 | 463.44 | 3.98738 | 17.04 |
| 14 | 24.07 | 134.8 | 3.69712 | 4.96 |
| 15 | 27.28 | 120.08 | 3.26919 | 4.42 |
| 16 | 31.50 | 53.54 | 2.8401 | 1.97 |
| 17 | 33.11 | 32.02 | 2.70593 | 1.18 |

In some embodiments of the present application, the TGA-DSC profile of the crystal form IV has an endothermic peak at 175 ± 2 °C.

In some embodiments of the present application, the TGA-DSC profile of the crystal form IV is substantially as shown in FIG. 2.

The present application also provides a method for preparing a crystal form IV of a compound of formula (I), comprising:

(a) adding the compound of formula (I) to a solvent and heating the mixture with stirring; and

(b) filtering the mixture to obtain a solid.

Wherein, the solvent is selected from organic solvents, preferably n-heptane; and the heating the mixture with stirring is conducted at a temperature of 40 to 60 °C, preferably 50 °C.

The present application also provides a further method for preparing a crystal form IV of a compound of formula (I), comprising:

(a) adding the compound of formula (I) to a solvent for dissolution; and

(b) filtering the mixture followed by slowly adding n-heptane dropwise to precipitate a solid.

Wherein, the solvent is selected from organic solvents, preferably 2-methyltetrahydrofuran.

In a second aspect of the present application, a crystal form VI of a compound of formula (I) is provided, which has characteristic peak(s) at 1, 2, 3, 4, 5, 6, 7 or 8 of 2θ values of 5.20°, 7.49°, 10.70°, 13.42°, 14.30°, 14.85°, 16.01°, and 18.66°, each with a 2θ error range of ± 0.2° in an X-ray powder diffraction pattern as measured using Cu-$K_\alpha$ radiation.

In some embodiments of the present application, the X-ray powder diffraction pattern of the crystal form VI has characteristic peaks at 2θ values of 5.20°, 7.49°, 10.70°, 13.42°, 14.30°, 14.85°, 16.01°, and 18.66°, each with a 2θ error range of ± 0.2°, as measured using Cu-$K_\alpha$ radiation.

In some embodiments of the present application, the X-ray powder diffraction pattern of the crystal form VI has characteristic peak(s) at 1, 2, 3, 4, 5, 6, 7, 8, 9, 10, 11, 12, 13, or 14 of 2θ values of 5.20°, 7.49°, 7.95°, 9.70°, 10.70°, 11.53°, 13.05°, 13.42°, 14.30°, 14.85°, 16.01°, 16.38°, 17.11°, and 18.66°, each with a 2θ error range of ± 0.2°, as measured using Cu-$K_\alpha$ radiation. In some embodiments of the present application, the X-ray powder diffraction pattern of the crystal form VI has characteristic peaks at 2θ values of 5.20°, 7.49°, 7.95°, 9.70°, 10.70°, 11.53°, 13.05°, 13.42°, 14.30°, 14.85°, 16.01°, 16.38°, 17.11°, and 18.66°, each with a 2θ error range of ± 0.2°, as measured using Cu-$K_\alpha$ radiation.

In some embodiments of the present application, the X-ray powder diffraction pattern of the crystal form VI has characteristic peak(s) at 1, 2, 3, 4, 5, 6, 7, 8, 9, 10, 11, 12, 13, 14, 15, 16, 17, 18, or 19 of 2θ values of 5.20°, 6.68°, 7.49°, 7.95°, 9.70°, 10.70°, 11.53°, 13.05°, 13.42°, 13.67°, 14.30°, 14.85°, 16.01°, 16.38°, 17.11°, 18.66°, 19.37°, 23.44°, 24.36°, each with a 2θ error range of ± 0.2°, as measured using Cu-$K_\alpha$ radiation.

In some embodiments of the present application, the X-ray powder diffraction pattern of the crystal form VI has characteristic peaks at 2θ values of 5.20°, 6.68°, 7.49°, 7.95°, 9.70°, 10.70°, 11.53°, 13.05°, 13.42°, 13.67°, 14.30°, 14.85°, 16.01°, 16.38°, 17.11°, 18.66°, 19.37°, 23.44°, and 24.36°, each with a 2θ error range of ± 0.2°, as measured using Cu-$K_\alpha$ radiation.

In some embodiments of the present application, the X-ray powder diffraction pattern of the crystal form VI is substantially as shown in FIG. 5.

In some embodiments of the present application, the X-ray powder diffraction peak analytical data of the crystal form VI is shown in Table 2.

Table 2 Analytical data of XRPD diffraction peaks of the crystal form VI of formula (I)

| No. | 2Theta (°) | Intensity (counts) | Interplanar spacing (Å) | Relative intensity (%) |
|---|---|---|---|---|
| 1 | 5.20 | 305.68 | 17.00698 | 23.5 |
| 2 | 6.68 | 152.18 | 13.23562 | 11.7 |
| 3 | 7.49 | 519.75 | 11.79843 | 39.97 |
| 4 | 7.95 | 216.21 | 11.12593 | 16.63 |
| 5 | 8.31 | 99.63 | 10.64596 | 7.66 |
| 6 | 9.11 | 145.35 | 9.70435 | 11.18 |
| 7 | 9.70 | 217.37 | 9.12163 | 16.71 |
| 8 | 10.70 | 1300.5 | 8.27102 | 100 |
| 9 | 11.53 | 176.78 | 7.67739 | 13.59 |
| 10 | 11.87 | 103.25 | 7.45593 | 7.94 |
| 11 | 13.05 | 214.17 | 6.78405 | 16.47 |
| 12 | 13.42 | 493.01 | 6.59934 | 37.91 |
| 13 | 13.67 | 343.28 | 6.47748 | 26.4 |
| 14 | 14.30 | 683.26 | 6.19436 | 52.54 |
| 15 | 14.85 | 402.95 | 5.96563 | 30.98 |
| 16 | 16.01 | 1273.55 | 5.53607 | 97.93 |
| 17 | 16.38 | 350.4 | 5.41123 | 26.94 |
| 18 | 16.63 | 346.77 | 5.33194 | 26.66 |
| 19 | 17.11 | 334.46 | 5.18217 | 25.72 |
| 20 | 18.66 | 367.33 | 4.75615 | 28.25 |
| 21 | 19.37 | 137.01 | 4.5818 | 10.54 |
| 22 | 21.69 | 129.82 | 4.09651 | 9.98 |
| 23 | 23.44 | 130.81 | 3.79527 | 10.06 |
| 24 | 24.36 | 183.12 | 3.65413 | 14.08 |
| 25 | 25.55 | 98.4 | 3.48679 | 7.57 |
| 26 | 28.84 | 72.79 | 3.09623 | 5.6 |
| 27 | 30.09 | 47.16 | 2.96967 | 3.63 |
| 28 | 32.17 | 31.15 | 2.78271 | 2.4 |
| 29 | 34.31 | 54.87 | 2.61373 | 4.22 |
| 30 | 36.93 | 36.33 | 2.43382 | 2.79 |
| 31 | 42.51 | 29.45 | 2.12651 | 2.26 |

In some embodiments of the present application, the TGA-DSC profile of the crystal form VI has an exothermic peak at 105 ± 2 °C and an endothermic peak at 177 ± 2 °C.

In some embodiments of the present application, the TGA-DSC profile of the crystal form VI is substantially as shown in FIG. 6.

The present application also provides a method for preparing a crystal form VI of a compound of formula (I), comprising:

(a) dissolving the compound of formula (I) in a solvent at elevated temperature; and

(b) filtering the mixture and slowly cooling to precipitate to a solid.

Wherein, the solvent is selected from the group consisting of methanol, acetonitrile, acetone, water, and a combination thereof; and the temperature of dissolving at elevated temperature is selected from 40 to 60 °C, preferably 50 °C.

The present application also provides a further method for preparing a crystal form VI of a compound of formula (I), comprising:

(a) stirring the compound of formula (I) in a solvent at room temperature; and

(b) filtering the mixture to obtain a solid.

Wherein, the solvent is selected from the group consisting of methanol, acetonitrile, acetone, water and a combination thereof.

The present application also provides a further method for preparing a crystal form VI of a compound of formula (I), comprising:

(a) dissolving the compound of formula (I) in a solvent;

(b) filtering the mixture followed by slowly cooling and stirring at a maintained temperature; and

(c) filtering the mixture and drying under vacuum.

Wherein, the solvent is selected from the group consisting of methanol, acetonitrile, acetone, water and a combination thereof.

The present application also provides a crystal form VIII of a compound of formula (I), which has characteristic peak(s) at 1, 2, 3, 4, 5, 6, or 7 of 2θ values of 8.97°, 13.51°, 16.20°, 18.05°, 18.56°, 20.94°, and 21.25°, each with a 2θ error range of ± 0.2° in an X-ray powder diffraction pattern as measured using Cu-$K_\alpha$ radiation.

In some embodiments of the present application, the X-ray powder diffraction pattern of the crystal form VIII has characteristic peaks at 2θ values of 8.97°, 13.51°, 16.20°, 18.05°, 18.56°, 20.94°, and 21.25°, each with a 2θ error range of ± 0.2°, as measured using Cu-$K_\alpha$ radiation.

In some embodiments of the present application, the X-ray powder diffraction pattern of the crystal form VIII has characteristic peak(s) at 1, 2, 3, 4, 5, 6, 7, 8, 9, or 10 of 2θ values of 8.97°, 13.51°, 15.51°, 16.20°, 18.05°, 18.56°, 19.94°, 20.94°, 21.25°, and 27.13°, each with a 2θ error range of ± 0.2°, as measured using Cu-$K_\alpha$ radiation.

In some embodiments of the present application, the X-ray powder diffraction pattern of the crystal form VIII has characteristic peaks at 2θ values of 8.97°, 13.51°, 15.51°, 16.20°, 18.05°, 18.56°, 19.94°, 20.94°, 21.25°, and 27.13°, each with a 2θ error range of ± 0.2°, as measured using Cu-$K_\alpha$ radiation.

In some embodiments of the present application, the X-ray powder diffraction pattern of the crystal form VIII has characteristic peak(s) at 1, 2, 3, 4, 5, 6, 7, 8, 9, 10, 11, 12, 13, or 14 of 2θ values of 8.97°, 10.75°, 13.51°, 15.51°, 16.20°, 17.88°, 18.05°, 18.56°, 19.94°, 20.94°, 21.25°, 22.67°, 25.07°, and 27.13°, each with a 2θ error range of ± 0.2°, as measured using Cu-$K_\alpha$ radiation.

In some embodiments of the present application, the X-ray powder diffraction pattern of the crystal form VIII has characteristic peaks at 2θ values of 8.97°, 10.75°, 13.51°, 15.51°, 16.20°, 17.88°, 18.05°, 18.56°, 19.94°, 20.94°, 21.25°, 22.67°, 25.07°, 27.13°, 25.07°, and 27.13°, each with a 2θ error range of ± 0.2°, as measured using Cu-$K_\alpha$ radiation.

In some embodiments of the present application, the X-ray powder diffraction pattern of the crystal form VIII is substantially as shown in FIG. 7.

In some embodiments of the present application, the X-ray powder diffraction peak analytical data of the crystal form VIII is shown in Table 3.

Table 3 Analytical data of XRPD diffraction peaks of the crystal form VIII of formula (I)

| No. | 2Theta (°) | Intensity (counts) | Interplanar spacing (Å) | Relative intensity (%) |
|---|---|---|---|---|
| 1 | 8.97 | 918.3 | 9.85784 | 26.38 |
| 2 | 10.75 | 419.02 | 8.23245 | 12.04 |
| 3 | 13.51 | 1546.08 | 6.55446 | 44.42 |
| 4 | 14.88 | 109.02 | 5.9542 | 3.13 |
| 5 | 15.51 | 739.49 | 5.71242 | 21.25 |
| 6 | 16.20 | 806.37 | 5.47078 | 23.17 |

(continued)

| No. | 2Theta (°) | Intensity (counts) | Interplanar spacing (Å) | Relative intensity (%) |
|---|---|---|---|---|
| 7 | 17.04 | 173.44 | 5.20344 | 4.98 |
| 8 | 17.88 | 623.62 | 4.96146 | 17.92 |
| 9 | 18.05 | 754.82 | 4.91389 | 21.69 |
| 10 | 18.56 | 2139.65 | 4.7818 | 61.47 |
| 11 | 18.93 | 171.3 | 4.68843 | 4.92 |
| 12 | 19.94 | 759.88 | 4.45209 | 21.83 |
| 13 | 20.94 | 3480.77 | 4.24296 | 100 |
| 14 | 21.25 | 1027.2 | 4.18099 | 29.51 |
| 15 | 21.65 | 371.86 | 4.10555 | 10.68 |
| 16 | 22.67 | 582.6 | 3.92323 | 16.74 |
| 17 | 23.12 | 66.36 | 3.84708 | 1.91 |
| 18 | 23.82 | 218.9 | 3.73494 | 6.29 |
| 19 | 24.08 | 427 | 3.69649 | 12.27 |
| 20 | 24.55 | 144.6 | 3.62615 | 4.15 |
| 21 | 25.07 | 594.15 | 3.55172 | 17.07 |
| 22 | 25.95 | 190.46 | 3.43366 | 5.47 |
| 23 | 27.13 | 766.11 | 3.28729 | 22.01 |
| 24 | 27.48 | 377.54 | 3.24534 | 10.85 |
| 25 | 29.91 | 164.64 | 2.98769 | 4.73 |
| 26 | 31.97 | 82.06 | 2.79926 | 2.36 |
| 27 | 34.52 | 111.94 | 2.5984 | 3.22 |
| 28 | 34.89 | 90.48 | 2.57128 | 2.6 |
| 29 | 39.35 | 56.31 | 2.28991 | 1.62 |
| 30 | 40.70 | 46.05 | 2.21706 | 1.32 |

In some embodiments of the present application, the TGA-DSC profile of the crystal form VIII has endothermic peaks at 124 ± 2 °C, 179 ± 2 °C and an exothermic peak at 128 ± 2 °C.

In some embodiments of the present application, the TGA-DSC profile of the crystal form VIII is substantially as shown in FIG. 8.

The present application also provides a crystal form X of a compound of formula (I), which has characteristic peak(s) at 1, 2, 3, 4, 5, 6, 7, or 8 of 2θ values of 11.61°, 15.66°, 17.49°, 18.36°, 19.51°, 21.28°, 23.60°, and 25.43°, each with a 2θ error range of ± 0.2° in an X-ray powder diffraction pattern as measured using Cu-$K_\alpha$ radiation.

In some embodiments of the present application, the X-ray powder diffraction pattern of the crystal form X has characteristic peaks at 2θ values of 11.61°, 15.66°, 17.49°, 18.36°, 19.51°, 21.28°, 23.60°, and 25.43°, each with a 2θ error range of ± 0.2°, as measured using Cu-$K_\alpha$ radiation.

In some embodiments of the present application, the X-ray powder diffraction pattern of the crystal form X has characteristic peak(s) at 1, 2, 3, 4, 5, 6, 7, 8, 9, 10, or 11 of 2θ values of 11.61°, 14.07°, 14.70°, 15.66°, 17.49°, 18.36°, 18.54°, 19.51°, 21.28°, 23.60°, and 25.43°, each with a 2θ error range of ± 0.2°, as measured using Cu-$K_\alpha$ radiation.

In some embodiments of the present application, the X-ray powder diffraction pattern of the crystal form X has characteristic peaks at 2θ values of 11.61°, 14.07°, 14.70°, 15.66°, 17.49°, 18.36°, 18.54°, 19.51°, 21.28°, 23.60°, and 25.43°, each with a 2θ error range of ± 0.2°, as measured using Cu-$K_\alpha$ radiation.

In some embodiments of the present application, the X-ray powder diffraction pattern of the crystal form X has characteristic peak(s) at 1, 2, 3, 4, 5, 6, 7, 8, 9, 10, 11, 12, 13, 14, 15, or 16 of 2θ values of 11.24°, 11.61°, 14.07°, 14.70°, 14.88°, 15.66°, 15.84°, 17.49°, 18.36°, 18.54°, 19.51°, 19.90°, 21.28°, 23.60°, 25.43°, and 27.67°, each with a 2θ error range of ± 0.2°, as measured using Cu-$K_\alpha$ radiation.

In some embodiments of the present application, the X-ray powder diffraction pattern of the crystal form X has characteristic peaks at 2θ values of 11.24°, 11.61°, 14.07°, 14.70°, 14.88°, 15.66°, 15.84°, 17.49°, 18.36°, 18.54°, 19.51°, 19.90°, 21.28°, 23.60, 25.43°, and 27.67°, each with a 2θ error range of ± 0.2°, as measured using Cu-$K_\alpha$ radiation.

In some embodiments of the present application, the X-ray powder diffraction pattern of the crystal form X is substantially as shown in FIG. 9.

In some embodiments of the present application, the X-ray powder diffraction peak analytical data of the crystal form X is shown in Table 4.

Table 4 Analytical data of XRPD diffraction peaks of the crystal form X of formula (I)

| No. | 2Theta (°) | Intensity (counts) | Interplanar spacing (Å) | Relative intensity (%) |
|---|---|---|---|---|
| 1 | 6.99 | 186.42 | 12.6485 | 4.02 |
| 2 | 9.10 | 140.78 | 9.72329 | 3.04 |
| 3 | 10.55 | 103.31 | 8.38499 | 2.23 |
| 4 | 11.24 | 486.44 | 7.8752 | 10.5 |
| 5 | 11.61 | 1121.93 | 7.62406 | 24.22 |
| 6 | 13.01 | 146.32 | 6.80592 | 3.16 |
| 7 | 14.07 | 604.59 | 6.29548 | 13.05 |
| 8 | 14.32 | 194.7 | 6.18516 | 4.2 |
| 9 | 14.70 | 604.09 | 6.02697 | 13.04 |
| 10 | 14.88 | 538.81 | 5.95339 | 11.63 |
| 11 | 15.06 | 384.27 | 5.88204 | 8.3 |
| 12 | 15.66 | 2325.42 | 5.66065 | 50.2 |
| 13 | 15.84 | 574.53 | 5.59546 | 12.4 |
| 14 | 17.49 | 2046.91 | 5.06965 | 44.19 |
| 15 | 18.36 | 1757.14 | 4.83156 | 37.93 |
| 16 | 18.54 | 887.24 | 4.78671 | 19.15 |
| 17 | 19.51 | 2728.33 | 4.54992 | 58.9 |
| 18 | 19.90 | 586.19 | 4.46233 | 12.65 |
| 19 | 20.11 | 408.75 | 4.41631 | 8.82 |
| 20 | 20.67 | 408.65 | 4.29639 | 8.82 |
| 21 | 21.28 | 4632.46 | 4.17572 | 100 |
| 22 | 21.71 | 331.38 | 4.09454 | 7.15 |
| 23 | 22.00 | 242.87 | 4.03967 | 5.24 |
| 24 | 22.38 | 379.89 | 3.97329 | 8.2 |
| 25 | 22.78 | 237.34 | 3.90376 | 5.12 |
| 26 | 23.60 | 1292.08 | 3.76986 | 27.89 |
| 27 | 24.27 | 143.99 | 3.66808 | 3.11 |
| 28 | 24.54 | 96.7 | 3.62717 | 2.09 |
| 29 | 25.43 | 1684.73 | 3.503 | 36.37 |
| 30 | 25.75 | 440.25 | 3.45948 | 9.5 |
| 31 | 26.28 | 155.48 | 3.39089 | 3.36 |
| 32 | 26.98 | 167.92 | 3.30441 | 3.62 |
| 33 | 27.37 | 432.23 | 3.25807 | 9.33 |
| 34 | 27.67 | 637.43 | 3.22374 | 13.76 |

(continued)

| No. | 2Theta (°) | Intensity (counts) | Interplanar spacing (Å) | Relative intensity (%) |
|---|---|---|---|---|
| 35 | 28.21 | 352.77 | 3.1639 | 7.62 |
| 36 | 29.28 | 66.95 | 3.0505 | 1.45 |
| 37 | 29.77 | 101.32 | 3.00155 | 2.19 |
| 38 | 30.45 | 92.09 | 2.93604 | 1.99 |
| 39 | 30.74 | 202.42 | 2.90896 | 4.37 |
| 40 | 31.89 | 74.58 | 2.80599 | 1.61 |
| 41 | 32.43 | 103.36 | 2.7609 | 2.23 |
| 42 | 32.88 | 52.83 | 2.7242 | 1.14 |
| 43 | 33.26 | 54.39 | 2.6938 | 1.17 |
| 44 | 33.80 | 129.17 | 2.65166 | 2.79 |
| 45 | 34.27 | 56.77 | 2.61634 | 1.23 |
| 46 | 35.88 | 75.19 | 2.50319 | 1.62 |
| 47 | 36.97 | 142.42 | 2.43134 | 3.07 |
| 48 | 38.43 | 38.07 | 2.3426 | 0.82 |
| 49 | 40.36 | 20.38 | 2.23483 | 0.44 |

In some embodiments of the present application, the TGA-DSC profile of the crystal form X has an endothermic peak at 179 ± 2 °C.

In some embodiments of the present application, the TGA-DSC profile of the crystal form X is substantially as shown in FIG. 10.

The present application also provides a pharmaceutically acceptable salt of a compound of formula (I) 2-(6-oxo-5-(trifluoromethyl)-1,6-dihydropyridin-3-yl)ethyl-4-(5-(trifluoromethyl)pyrimidin-2-yl) piperazine-1-carboxylate,

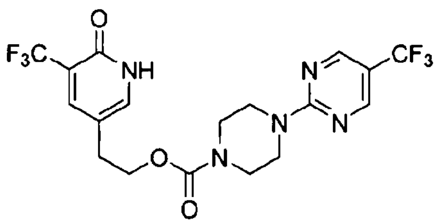

(I) .

In some embodiments of the present application, the pharmaceutically acceptable salt of the compound of formula (I) is a sodium salt.

In some embodiments of the present application, the pharmaceutically acceptable salt of the compound of formula (I) is a sodium salt having the following structure:

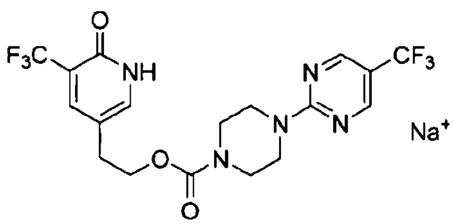

(I-1) .

The present application also provides a crystalline form of the pharmaceutically acceptable sodium salt of formula (I-1).

In some embodiments of the present application, the crystalline form of the pharmaceutically acceptable sodium

salt of formula (I-1) is the crystal form I, which has characteristic peak(s) at 1, 2, 3, 4, or 5 of 2θ values of 15.99°, 17.16°, 17.29°, 17.70°, and 23.24°, each with a 2θ error range of ± 0.2° in the X-ray powder diffraction pattern as measured using Cu-$K_\alpha$ radiation.

In some embodiments of the present application, the X-ray powder diffraction pattern of the crystal form I has characteristic peaks at 2θ values of 15.99°, 17.16°, 17.29°, 17.70°, and 23.24°, each with a 2θ error range of ± 0.2°, as measured using Cu-$K_\alpha$ radiation.

In some embodiments of the present application, the X-ray powder diffraction pattern of the crystal form I has characteristic peak(s) at 1, 2, 3, 4, 5, 6, 7, or 8 of 2θ values of 8.99°, 15.99°, 17.16°, 17.29°, 17.70°, 21.39°, 22.92°, and 23.24°, each with a 2θ error range of ± 0.2°, as measured using Cu-$K_\alpha$ radiation.

In some embodiments of the present application, the X-ray powder diffraction pattern of the crystal form I has characteristic peaks at 2θ values of 8.99°, 15.99°, 17.16°, 17.29°, 17.70°, 21.39°, 22.92°, and 23.24°, each with a 2θ error range of ± 0.2°, as measured using Cu-$K_\alpha$ radiation.

In some embodiments of the present application, the X-ray powder diffraction pattern of the crystal form I has characteristic peak(s) at 1, 2, 3, 4, 5, 6, 7, 8, 9, or 10 of 2θ values of 8.99°, 12.92°, 15.99°, 17.16°, 17.29°, 17.70°, 20.98°, 21.39°, 22.92°, and 23.24°, each with a 2θ error range of ± 0.2°, as measured using Cu-$K_\alpha$ radiation.

In some embodiments of the present application, the X-ray powder diffraction pattern of the crystal form I has characteristic peaks at 2θ values of 8.99°, 12.92°, 15.99°, 17.16°, 17.29°, 17.70°, 20.98°, 21.39°, 22.92°, and 23.24°, each with a 2θ error range of ± 0.2°, as measured using Cu-$K_\alpha$ radiation.

In some embodiments of the present application, the X-ray powder diffraction pattern of the crystal form I is substantially as shown in FIG. 13.

In some embodiments of the present application, the X-ray powder diffraction peak analytical data of the crystal form I is shown in Table 5.

Table 5 Analytical data of XRPD diffraction peaks of the crystal form I

| No. | 2Theta (°) | Intensity (counts) | Interplanar spacing (Å) | Relative intensity (%) |
|---|---|---|---|---|
| 1 | 8.99 | 1324.07 | 9.8384 | 24.27 |
| 2 | 9.59 | 269.06 | 9.22672 | 4.93 |
| 3 | 10.04 | 742.59 | 8.81237 | 13.61 |
| 4 | 10.33 | 565.77 | 8.55995 | 10.37 |
| 5 | 12.20 | 346.85 | 7.25439 | 6.36 |
| 6 | 12.92 | 1047.75 | 6.85187 | 19.21 |
| 7 | 13.38 | 275.14 | 6.6182 | 5.04 |
| 8 | 14.40 | 394.75 | 6.14994 | 7.24 |
| 9 | 14.83 | 520 | 5.97508 | 9.53 |
| 10 | 15.28 | 109.7 | 5.79781 | 2.01 |
| 11 | 15.99 | 1706.27 | 5.5419 | 31.28 |
| 12 | 16.71 | 867.15 | 5.30505 | 15.9 |
| 13 | 17.16 | 2743.62 | 5.16834 | 50.29 |
| 14 | 17.29 | 1728.59 | 5.13001 | 31.69 |
| 15 | 17.70 | 3560.94 | 5.01128 | 65.28 |
| 16 | 18.05 | 1012.72 | 4.91412 | 18.56 |
| 17 | 18.20 | 513.61 | 4.87318 | 9.41 |
| 18 | 18.85 | 717.92 | 4.70801 | 13.16 |
| 19 | 19.90 | 1016.72 | 4.46196 | 18.64 |
| 20 | 20.18 | 413.99 | 4.40082 | 7.59 |
| 21 | 20.98 | 1032.96 | 4.2344 | 18.94 |
| 22 | 21.39 | 1111.79 | 4.15478 | 20.38 |
| 23 | 21.58 | 603.47 | 4.11846 | 11.06 |

(continued)

| No. | 2Theta (°) | Intensity (counts) | Interplanar spacing (Å) | Relative intensity (%) |
|---|---|---|---|---|
| 24 | 22.55 | 624.35 | 3.94265 | 11.44 |
| 25 | 22.92 | 1154.72 | 3.88053 | 21.17 |
| 26 | 23.24 | 5455.2 | 3.82828 | 100 |
| 27 | 23.96 | 417.06 | 3.71412 | 7.65 |
| 28 | 25.02 | 611.63 | 3.55877 | 11.21 |
| 29 | 25.21 | 709.3 | 3.53207 | 13 |
| 30 | 25.48 | 901.56 | 3.4954 | 16.53 |
| 31 | 25.98 | 322.43 | 3.43033 | 5.91 |
| 32 | 26.53 | 138.03 | 3.36048 | 2.53 |
| 33 | 27.60 | 500.06 | 3.23208 | 9.17 |
| 34 | 28.09 | 148.04 | 3.17658 | 2.71 |
| 35 | 29.10 | 177.28 | 3.0689 | 3.25 |
| 36 | 29.63 | 348.82 | 3.01519 | 6.39 |
| 37 | 30.67 | 260.28 | 2.91521 | 4.77 |
| 38 | 31.88 | 124.71 | 2.80676 | 2.29 |
| 39 | 32.73 | 177.91 | 2.73583 | 3.26 |
| 40 | 33.03 | 132.94 | 2.71224 | 2.44 |
| 41 | 33.71 | 247.14 | 2.65904 | 4.53 |
| 42 | 34.96 | 213.24 | 2.56692 | 3.91 |
| 43 | 35.66 | 67.42 | 2.51806 | 1.24 |

In some embodiments of the present application, the TGA-DSC profile of the crystal form I has an exothermic peak at 241 ± 2 °C.

In some embodiments of the present application, it is characterized in that the TGA-DSC profile of the crystal form I is substantially as shown in FIG. 14.

The present application also provides a method for preparing a sodium salt of a compound of formula (1-1), comprising: adding the compound of formula (I) to a solvent to dissolve, and filtering; and adding sodium ethoxide solution dropwise to form the salt.

In some embodiments of the present application, the solvent used in the above salt formation reaction is selected from organic solvents, preferably ethanol.

The present application also provides a method for preparing a crystal form I of a pharmaceutically acceptable sodium salt of formula (I-1), comprising:

(a) adding the compound of formula (I-1) to a solvent 1 to dissolve; and

(b) filtering and followed by stirring at room temperature, slowly adding a solvent 2 dropwise to precipitate a solid.

In some embodiments of the present application, the solvent 1 is selected from organic solvents, preferably tetrahydrofuran; and the solvent 2 is selected from organic solvents, preferably n-heptane or methyl isobutyl ketone.

The present application also provides a pharmaceutical composition comprising a pharmaceutically acceptable salt or any one of the above-specified crystal forms of a compound of formula (I), and one or more pharmaceutically acceptable carrier(s). Preferably, the pharmaceutically acceptable salt or any one of the above-specified crystal forms of the compound of formula (I) is in a therapeutically effective amount. The pharmaceutical composition comprising a pharmaceutically acceptable salt or any one of the above-specified crystal forms of the compound of formula (I) according to the present application may comprise the pharmaceutically acceptable salt, crystal form IV, crystal form VI, crystal form VIII, crystal form X, or crystal form I of the compound of formula (I) as specified above, individually, or may comprise two or more of the pharmaceutically acceptable salt, crystal form IV, crystal form VI, crystal form VIII, crystal form X, and crystal

form I of the compound of formula (I) as specified above.

The present application also provides use of a pharmaceutically acceptable salt, any one of the above-specified crystal forms of a compound of formula (I), or the pharmaceutical composition comprising any one of the above-specified crystal forms of a compound of formula (I) in the preparation of a drug for treating and/or preventing tumors. Any one of the above-specified crystal forms in the present application refers to the crystal form IV, crystal form VI, crystal form VIII, crystal form X, or crystal form I as specified above.

The present application also provides a method for treating and/or preventing a tumor, comprising administering a therapeutically and/or prophylactically effective amount of a pharmaceutically acceptable salt, or any one of the above-specified crystal forms of a compound of formula (I) to a patient.

In some embodiments of the present application, in the above-specified pharmaceutical composition, the use in the preparation of a drug, and the method for treating, and the pharmaceutically acceptable salt is a sodium salt.

In some embodiments of the present application, in the above-specified pharmaceutical composition, the use in the preparation of a drug, and the method for treating, and the pharmaceutically acceptable salt is a sodium salt having the following structure:

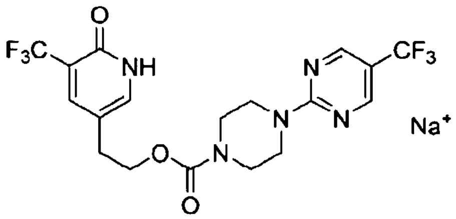

(I-1) .

In some embodiments of the present application, the above-specified pharmaceutically acceptable sodium salt of formula (I-1) is in a crystalline form.

In some embodiments of the present application, the crystalline form of the above-specified pharmaceutically acceptable sodium salt of formula (I-1) is the crystal form I.

In some embodiments of the present application, a formation of the tumor is associated with PARP; preferably, the PARP is PARP7.

The crystal form provided in the present application has better chemical and physical stability as well as lower hygroscopicity, which is less affected by temperature, humidity and light, and facilitates storage and formulation development.

## Brief Description of the Drawings

The figures illustrated herein are used to provide a further understanding of the present application and form part of the present application, and the exemplary examples of the present application and their description are used to explain the present application and do not constitute an undue limitation of the present application.

FIG. 1 is an XRPD spectrum showing a crystal form IV of a compound of formula (I);

FIG. 2 is a TGA-DSC spectrum showing a crystal form IV of a compound of formula (I);

FIG. 3 is a DVS spectrum showing a crystal form IV of a compound of formula (I);

FIG. 4 is a comparison of crystal form spectrum showing a crystal form IV of a compound of formula (I) before and after the DVS test;

FIG. 5 is an XRPD spectrum showing a crystal form VI of a compound of formula (I);

FIG. 6 is a TGA-DSC spectrum showing a crystal form VI of a compound of formula (I);

FIG. 7 is a XRPD spectrum showing a crystal form VIII of a compound of formula (I);

FIG. 8 is a TGA-DSC spectrum showing a crystal form VIII of a compound of formula (I);

FIG. 9 is an XRPD spectrum showing a crystal form X of a compound of formula (I);

FIG. 10 is a TGA-DSC spectrum showing a crystal form X of a compound of formula (I);

FIG. 11 is a DVS spectrum of showing a crystal form X of a compound of formula (I);

FIG. 12 is a comparison of crystal form spectrum of a crystal form X of a compound of formula (I) before and after the DVS test;

FIG. 13 is an XRPD spectrum showing a crystal form I of a pharmaceutically acceptable salt of a compound of formula (I);

FIG. 14 is a TGA-DSC spectrum showing a crystal form I of a pharmaceutically acceptable salt of a compound of formula (I);

FIG. 15 is a DVS spectrum showing a crystal form I of a pharmaceutically acceptable salt of a compound of formula (I).

## Detailed Description

The present application will be described in detail by way of examples. It does not imply any unfavorable limitation of the present application. The compounds of the present application can be prepared by a variety of synthesis methods known to those skilled in the art, including the specific embodiments recited below, embodiments formed by combining thereof with other chemical synthesis methods, and the equivalent replacement methods known to those skilled in the art, and preferred embodiments include, but are not limited to, the examples of the present application. Various variations and modifications for the specific embodiments of the present application without departing from the spirit and scope of the present application will be apparent to those skilled in the art.

In the absence of special instructions, all reactions of the present application are carried out under continuous magnetic stirring under dry nitrogen or argon atmosphere, the solvent is a dry solvent, and the temperature of the reaction is in degrees Celsius or °C. If not otherwise specified, room temperature refers to 25 ± 5 °C.

### Descriptions and definitions

Unless otherwise indicated, the following terms and phrases used herein are intended to have the meanings set forth below. A particular term or phrase should not be regarded as indefinite or unclear without special definition, but should be understood in its ordinary meaning.

The term "composition" means a product comprising a specified amount of an ingredient, and a product derived directly or indirectly from a combination of specified amounts of each of the specified ingredients.

The term "pharmaceutically acceptable carrier" means a medium generally acceptable in the art for the delivery of a biologically active reagent to an animal, particularly a mammal, including, for example, adjuvants, excipients or vehicles, such as diluents, preservatives, fillers, flow modifiers, disintegrators, humectants, emulsifiers, suspension agents, sweeteners, flavoring agents, fragrances, antibacterial agents, antifungal agents, lubricants and dispersing agents, depending on the way of administration and the nature of dosage forms. The pharmaceutically acceptable carrier is formulated within the scope of common knowledge of those skilled in the art based on a variety of factors.

The term "therapeutically effective amount" means that the compound of the present application or a pharmaceutically acceptable salt thereof is present in a sufficient amount to treat the disorder with a reasonable effect/risk ratio applicable to any medical therapy and/or prophylaxis. It should be recognized that the total daily dosage of a compound of formula (I), or a pharmaceutically acceptable salt or a composition thereof of the present application is determined by an attending physician within the bounds of sound medical judgment. For any specific patient, the specific therapeutically effective dosage level shall be based on a variety of factors, including the disorder being treated and the severity of the disorder; the activity of the specific compound used; the specific composition used; age, body weight, general health, gender, and diet of the patient; the time of administration, route of administration, and rate of excretion of the specific compound used; the duration of treatment; the medicament used in combination with or simultaneously with the specific compounds used; and similar factors well known in the medical field.

It is well known in the art that X-ray powder diffraction patterns have one or more measurement errors based on small variations in measurement conditions. The structure of the crystalline, the crystal, or the crystal form disclosed or claimed by the present application may exhibit similar, but not identical analytical properties within a reasonable margin of error based on test conditions, purity, equipment, and other often several variables known to those skilled in the art. For example, the angle of diffraction ($2\theta$) in X-ray powder diffraction typically yields an error within a range of ± 0.20°, therefore, the present application includes not only crystalline with identical angles of diffraction in X-ray powder diffraction, but also crystalline with identical angles of diffraction within an error range of ± 0.20°. The crystalline form of the compound of

formula (I) of the present application is not limited to crystals having an X-ray powder diffraction pattern identical to that shown in the figures, and any crystals having substantially the same X-ray powder diffraction pattern as that shown in the figures are within the scope of the present application.

In the present application, organic solvents include ester solvents or esters, alcohol solvents or alcohols, aliphatic hydrocarbon solvents or aliphatic hydrocarbons, ketone solvents or ketones, and ether solvents or ethers.

In the present application, examples of ester solvents or esters include, but are not limited to, dichloromethane, methyl acetate, ethyl acetate, n-propyl acetate, and isopropyl acetate.

In the present application, examples of alcohol solvents or alcohols include, but are not limited to, methanol, ethanol, propanol, isopropanol, and n-butanol.

In the present application, examples of aliphatic hydrocarbon solvents or aliphatic hydrocarbons include, but are not limited to, n-pentane, i-pentane, n-hexane, n-heptane, and n-octane.

In the present application, ketone solvents or ketones include, but are not limited to, acetone, methyl ethyl ketone, methyl butyl ketone, and methyl isobutyl ketone.

In the present application, ether solvents or ethers include, but are not limited to, ethyl ether, isopropyl ether, ethylene oxide, and methyl tert-butyl ether.

In the present application, alcohol/aliphatic hydrocarbon means a mixed solvent of alcohol solvents and aliphatic hydrocarbon solvents.

In the present application, the X-ray powder diffraction pattern of the crystal form IV has characteristic peak(s) at 1, 2, 3, 4 or 5 of 2θ values of 14.12, 15.56, 17.32, 18.06, 22.29, each with a 2θ error range of ± 0.2°, wherein 1, 2, 3, 4 or 5 means that any crystal forms having 1, 2, 3, 4 or 5 characteristic peak(s) identical to those of the crystal form I are included in the scope of the present application.

By "substantially the same X-ray powder diffraction pattern as that shown in the figures" as used in the present application, it should be understood that "substantially the same" in this context is also intended to indicate that the 2θ angle values of the X-ray powder diffraction patterns may vary slightly due to the inherent experimental variations accompanying these measurements, both being the same form of the crystal.

It should be understood that different types of equipment or different test conditions may give slightly different DSC profiles and endothermic transition temperature readings, and that DSC data can reflect changes in the morphology of a substance, with strong absorbance peaks indicating dehydration or desolvation, or transcrystallization, or melting, etc., and that when reflecting a molten state, the corresponding temperature is commonly understood to be a melting point of the substance. This value will be affected by the purity of the compound, the weight of the sample, the heating rate, the particle size and the calibration and maintenance of the test equipment. It will be understood by those skilled in the art that the temperature at which a substance transforms from a solid to a liquid state is usually a range of temperatures rather than a fixed point value, so that the temperature corresponding to the endothermic peak or the melting point of the substance may be characterized either by an onset value or a peak value or by other reasonable values. The maximum endothermic transition temperature of the crystal form may be within the range of ± 5.0 °C, preferably ± 2.0 °C, of the specific values disclosed above.

The present application also analyzes the relationship between the degree of decomposition or sublimation or evaporation of the crystal form (weight loss) and temperature, using thermo-gravimetric analysis (TGA). It should be understood that the values obtained for the same crystal form are subject to some error due to the purity of the sample, the particle size, different types of equipment, different test methods, etc. The temperature at which decomposition or sublimation or evaporation of a crystal form occurs can be within the range of ± 3.0 °C, such as ± 2.0 °C, of the specific values disclosed above.

The "stability" of the crystal form includes "chemical stability" and/or "physical stability". "Chemical stability" refers to the degree of degradation reaction of the crystal form under certain conditions of temperature, humidity, and light. "Chemical stability" reflects the stability of the crystal form under storage conditions. "Physical stability" refers to the degree of transformation of the crystal form into solid form under certain specific conditions, for example, transformation into another crystal form under the condition of high temperature, high humidity, grinding, tabletting, desolventization, or adsorption of solvents. Thus, "physical stability" can reflect the stability of the crystal form during the use of formulations and other processes in a certain extent.

Regarding the characterization of hygroscopicity and the definition of hygroscopic weight gain (General Chapter 9103 Guidelines for Hygroscopicity Test of Drugs, Chinese Pharmacopoeia 2020 Edition):

Deliquescent: absorbing sufficient amount of water to form a liquid;

Extremely hygroscopic: hygroscopic weight gain of not less than 15.0%;

Hygroscopic: hygroscopic weight gain of less than 15.0% but not less than 2.0%;

Slightly hygroscopic: hygroscopic weight gain less than 2.0% but not less than 0.2%;

Non- or almost non-hygroscopic: hygroscopic weight gain less than 0.2%.

The hygroscopicity directly affects the physicochemical stability of drugs, and high hygroscopicity can easily cause chemical degradation and transformation of the crystal form. In addition, high hygroscopicity will reduce the fluidity of the drug, thus affecting the processing of the drug. Moreover, drugs with high hygroscopicity need to maintain low humidity during the generation and storage process, which puts forward higher requirements for the generation and requires high costs. More importantly, high hygroscopicity can easily cause changes in the content of active ingredients in drugs, affecting the quality of drugs.

Bulk drugs and formulations will encounter high temperature and high humidity conditions caused by seasonal differences, climatic differences in different regions and weather factors during storage, transportation and production. Crystal forms with better stability are helpful to avoid the effect of deviation from the storage conditions on the label on the quality of the drug.

Transformation of the crystal form can lead to changes in the absorption of drugs, affecting bioavailability, and good chemical stability ensures that there are substantially no impurities generated during storage. Good physical and chemical stability of the crystal form ensures that the quality of the bulk drugs and formulations is always controllable, and minimizes the changes in the quality of the drugs due to changes in the crystal form or generation of impurities, changes in the bioavailability, and even the toxic side effects of the drugs.

In the present application, the "stirring" is accomplished by conventional methods in the field, such as magnetic stirring or mechanical stirring, with a stirring speed of 50 to 1800 rpm, wherein the magnetic stirring is preferably 300 to 900 rpm and the mechanical stirring is preferably 100 to 300 rpm.

The "drying" may be carried out at room temperature or at a higher temperature. The drying temperature is room temperature to about 60 °C, or to 50 °C, or to 40 °C. The drying time can be 2 to 48 hours, or overnight. Drying is carried out in a fume hood, a blast oven, or a vacuum oven.

The crystalline structures of the present application can be prepared by a variety of methods including crystallization or recrystallization from a suitable solvent, sublimation, growth from a melt, solid state transformation from another phase, crystallization from supercritical fluids, and jet spraying, and the like. Techniques for crystallization or recrystallization of crystalline structures from solvent mixtures include solvent evaporation, lowering the temperature of the solvent mixture, priming of the supersaturated solvent mixture of the molecule and/or salt, lyophilization of the solvent mixture, addition of counter-solvents to the solvent mixture, and the like.

The reaction temperature is in degrees Celsius or °C. If not otherwise indicated, room temperature means 25 $\pm$ 5 °C.

If not otherwise specified, formula (I), the compound of formula (I), or a free acid of the present application all refer to 2-(6-oxo-5-(trifluoromethyl)-1,6-dihydropyridin-3-yl)ethyl-4-(5-(trifluoromethyl)pyrimidin-2-yl) piperazine-1-carboxylate:

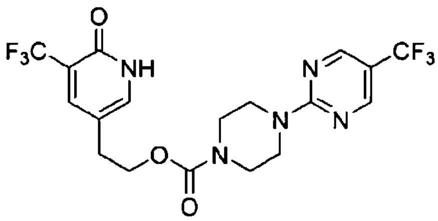

(I) .

If not otherwise specified, the crystal form of the present application all refer to the crystal form of the sodium salt of the compound of formula (I), i.e., the crystal form of formula (1-1).

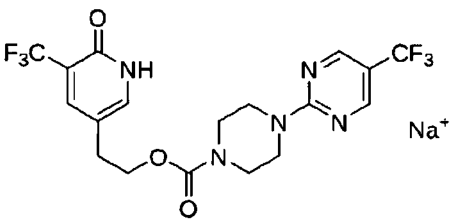

(I-1) .

The compound of the present application can be prepared by a variety of synthetic methods known to those skilled in the art, including the specific embodiments recited below, embodiments formed by combinations thereof with other chemical synthesis methods, and the equivalents known to those skilled in the art, and preferred embodiments include, but are not limited to, the examples of the present application.

In the examples of the present application, the naming of the compound is transformed through the structure of the compound with the aid of Chemdraw. If there is inconsistency between the compound name and the compound structure, it can be determined with the aid of synthesizing the relevant information and the reaction route; if it is not possible to confirm by other means, the given structural formula of the compound shall prevail.

The preparation methods of some of the compounds in the present application cite the preparation methods of the aforementioned similar compounds. Those in the field should know that when using or referring to the use of the preparation methods cited therein, the feed ratio of the reactants, the reaction solvent, the reaction temperature, etc., may be appropriately adjusted according to the differences in the reactants.

**Instrumentation and analytical methods:**

1. X-ray powder diffraction (XRPD)

The solid samples were analyzed by X-ray powder diffractometer (X'Pert PRO), an appropriate amount of fine powder of the tested sample was taken and placed in the groove of the sample holder and pressed with a glass sheet to form a flat and dense plane. The parameters of the XRPD measurement are shown in Table 6.

Table 6 Parameters of the XRPD test

| | |
|---|---|
| Instrument | PANalytical, X'Pert PRO |
| Radiation source | Cu target |
| Scan angle range | 3 to 45° (2θ) |
| Scan rate | 8°/min |
| Tube voltage/current | 40 KV/40 mA |
| Divergence slit | 1/8° |

2. Thermogravimetric analysis (TGA)

Thermogravimetric analysis of the solids was carried out using a thermogravimetric analyzer from TA Instrument. Approximately 1 to 5 mg of the sample was placed in a tared aluminum sample pan, the sample was heated according to the parameters listed in Table 7, and the data were analyzed using TRIOS.

Table 7 Parameters of the TGA analysis method

| | |
|---|---|
| Instrument | TA, Discovery TGA 550 |
| Sample pan | Alumina pan, open |
| Temperature range | 25 to 400 °C |
| Heating rate | 10 °C/min |
| Purge gas | Nitrogen |
| Flow rate | Balance chamber: 10 mL/min |
| | Sample chamber: 200 mL/min |

3. Differential scanning calorimetry (DSC) analysis

DSC analysis of the solids was carried out using differential scanning calorimeter from TA Instrument. Approximately 1 to 3 mg of the sample was accurately weighed and placed in a perforated aluminum sample pan, the sample was heated according to the parameters listed in Table 8, and the data were analyzed using TA Universal Analysis.

Table 8 Parameters of the DSC analysis method

| | |
|---|---|
| Instrument | TA, DSC Q2000 |
| Sample pan | Aluminum pan |
| Temperature range | 25 °C to 300 °C |
| Heating rate | 10 °C/min |
| Purge gas | Nitrogen |
| Flow rate | 50 mL/min |

Alternatively, thermogravimetric-differential scanning calorimetry analysis of the solids was carried out using a simultaneous thermal analyzer from Mettler Toledo. An appropriate amount of the test material was taken with a small spoon and placed in a crucible to make a uniform spread, its weight was weighed, the sample was heated according to the parameters listed in Table 9, and the data were analyzed using STARe.

Table 9 Parameters of TGA-DSC analytical method

| | |
|---|---|
| Instrument | Mettler Toledo, TGA/DSC1 |
| Sample pan | Aluminum crucible |
| Temperature range | 25 °C to 400 °C |
| Heating rate | 10 °C/min |
| Purge gas | Nitrogen |
| Flow rate | Balance chamber: 20 mL/min |
| | Sample chamber: 50 mL/min |

4. Dynamic vapor sorption (DVS) analysis

The moisture adsorption of the sample was determined using a dynamic vapor sorption instrument from DVS Intrinsic. The sample was placed in a tared sample basket and weighed by the instrument automatically and analyzed according to the parameters in Table 10.

Table 10 Parameters of the DVS analytical method

| | |
|---|---|
| Instrument | SMS, DVS Intrinsic |
| dm/dt | 0.002%/min |
| Sample weight | 10 to 30 mg |
| Drying/test temperature | 40 °C/ 25 °C |
| Cycle | Full cycle |
| Equilibrium time | 10 to 360 min |
| Data storage rate | 1 min |
| Gas and flow rate | $N_2$, 200 sccm |
| Post-run flow rate | 0 sccm |
| Step size | 10% RH |
| Method | Adsorption: 0, 10, 20, 30, 40, 50, 60, 70, 80, 90 |
| | Desorption: 80, 70, 60, 50, 40, 30, 20, 10, 0 |

5. Hydrogen nuclear magnetic resonance spectroscopy ($^1$H-NMR)

NMR was determined using an NMR instrument from Bruker AVANCE NEO 400 with deuterated dimethyl sulfoxide (DMSO-$d_6$) as the determined solvent.

6. High performance liquid chromatography (HPLC)

HPLC was used for the determination of the samples using Waters e2695 high performance liquid chromatography instrument.

7. Karl Fischer moisture titration (KF) test method:

(1) Instrumentation

Analytical balance (Sartorius, MSE125P), Karl Fischer moisture meter (Beijing Xianfengweifeng Technology Co., Ltd., ZDJ400).

(2) Reagents and test solution

Water, Fischer's test solution, and methanol.

(3) Operation procedure

The test was according to the moisture determination method (General Chapter 0832 Method I 1, Volume IV, Chinese Pharmacopoeia 2020 Edition).

Calibration: an appropriate amount of water was taken, precisely weighed, and calibrated 3 times to determine the titer of the volumetric solution used; and then the recovery rate was verified by back-titration, and the results of recovery rate should be in the range of 97.5%-102.5%.

Detection: 0.1 to 0.2 g of the sample was weighed, added to the titration cup containing methanol, and measured after completely dissolved.

(4) Calculation formula:

Moisture content in the tested sample (%) = $(A \times F) / (W \times 1000) \times 100\%$;

wherein: A: the volume of Fischer's test solution consumed by the tested sample (mL);

F: weight equivalent to water per 1 mL of Fischer's test solution (mg);

W: weight of the tested sample (g).

8. Gas chromatography (GC)

GC was determined using an Agilent 7890A/B-7697A Agilent gas chromatograph for chromatographic analysis of the samples.

The present application is further described below by specific examples.

**Example 1 Preparation method of the compound of formula (I)**

2-(6-oxo-5-(trifluoromethyl)-1,6-dihydropyridin-3-yl)ethyl-4-(5-(trifluoromethyl)pyrimidin -2-yl)piperazine-1-carboxylate (compound of formula I)

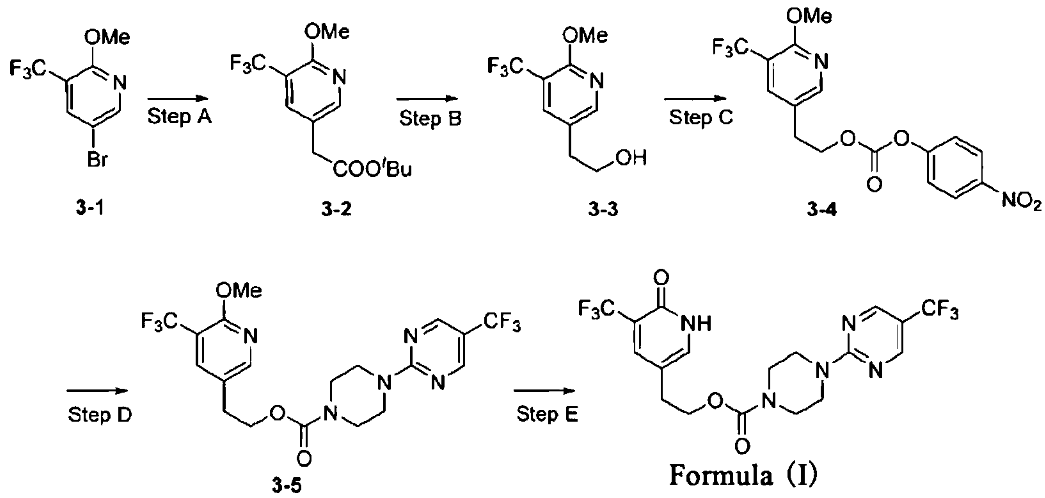

3-1 3-2 3-3 3-4 3-5 Formula (I)

Step A: Activated zinc powder (0.8 g, 12 mmol) was added to dry tetrahydrofuran (20 mL) under nitrogen protection, tert-butyl bromoacetate (1.5 g, 7.6 mmol) was added dropwise at reflux, and then the mixture was refluxed for 1 hour after initiation. 5-Bromo-2-methoxy-3-(trifluoromethyl)pyridine (300 mg, 1.2 mmol) was added to the above reaction solution, followed by the addition of catalytic amounts of tri(dibenzylideneacetone)dipalladium ($Pd_2(dba)_3$) and 2-dicyclohexylphosphino-2',4',6'-triisopropylbiphenyl (X-Phos). Subsequently, the reaction solution was refluxed and stirred overnight. After cooling to room temperature, the reaction solution was quenched with water, and the aqueous phase was extracted with ethyl acetate (100 mL x 3 times). The organic phases were combined, first washed with saturated brine (30 mL x 2 times), then dried with anhydrous sodium sulfate, filtered, and concentrated under reduced pressure. The resulting residue was purified by silica gel column chromatography to obtain 350 mg of tert-butyl 2-(6-methoxy-5-(trifluoromethyl)pyridin-3-yl)acetate (3-2).

MS (ESI) M/Z: 292.1 $[M+H]^+$.

$^1H$ NMR (400 MHz, $CDCl_3$) δ 8.18 (d, *J* = 2.2 Hz, 1H), 7.82 (d, *J* = 2.4 Hz, 1H), 4.03 (s, 3H), 3.50 (s, 2H), 1.45 (s. 9H).

Step B: tert-butyl 2-(6-methoxy-5-(trifluoromethyl)pyridin-3-yl)acetate (200 mg, 0.7 mmol) was dissolved in tetrahydrofuran (10 mL), lithium aluminum hydride (60 mg, 1.5 mmol) was added, and the reaction solution was heated and stirred for 5 hours at reflux. After cooling to room temperature, the reaction solution was quenched with saturated ammonium chloride solution, the aqueous phase was extracted with ethyl acetate (50 mL x 3 times). The organic phases were combined, first washed with saturated brine (20 mL x 2 times), then dried with anhydrous sodium sulfate, filtered and concentrated under reduced pressure. The resulting residue was purified by silica gel column chromatography to obtain 104 mg of 2-(6-methoxy-5-(trifluoromethyl)pyridin-3-yl)ethan-1-ol (3-3).

MS (ESI) M/Z: 221.9 $[M+H]^+$.

Step C: 2-(6-methoxy-5-(trifluoromethyl)pyridin-3-yl)ethan-1-ol (75 mg, 0.34 mmol) was dissolved in *N,N*-dimethylformamide (2 mL), and bis(p-nitrophenyl) carbonate (206 mg, 0.68 mmol) and *N,N*-diisopropylethylamine (90 mg, 0.68 mmol) were added. The reaction solution was heated to 80 °C and stirred for 2 hours. After cooling to room temperature, the reaction solution was quenched with water, and extracted with ethyl acetate (20 mL x 3 times). The organic phase was washed with saturated brine (20 mL), then dried with anhydrous sodium sulfate, filtered, and finally concentrated under reduced pressure. The resulting residue was purified by silica gel column chromatography to obtain 130 mg of 2-(6-methoxy-5-(trifluoromethyl)pyridin-3-yl)ethyl (4-nitrophenyl) carbonate (3-4), which was used directly in the next step of the reaction.

MS (ESI) M/Z: 386.9 $[M+H]^+$.

Step D: At room temperature, 2-(6-methoxy-5-(trifluoromethyl)pyridin-3-yl)ethyl (4-nitrophenyl) carbonate (130 mg, 0.34 mmol) was dissolved in *N,N*-dimethylformamide (2 mL), and 2-(piperazin-1-yl)-5-(trifluoromethyl)pyrimidine (80 mg, 0.34 mmol) and *N,N*-diisopropylethylamine (90 mg, 0.68 mmol) were added. The reaction solution was heated to 80 °C and stirred for 2 hours. After cooling to room temperature, the reaction solution was quenched with saturated ammonium chloride solution. The aqueous phase was extracted with dichloromethane (50 mL x 2 times), washed with saturated brine (20 mL), then dried with anhydrous sodium sulfate, filtered, and finally concentrated under reduced pressure to obtain 205 mg of 2-(6-methoxy-5-(trifluoromethyl)pyridin-3-yl)ethyl 4-(5-(trifluoromethyl)pyrimidin-2-yl)piperazine-1-carboxylate (3-5), which was used directly in the next step of the reaction.

MS (ESI) M/Z: 480.2 $[M+H]^+$.

Step E: At room temperature, 2-(6-methoxy-5-(trifluoromethyl)pyridin-3-yl)ethyl 4-(5-(trifluoromethyl)pyrimidin-2-yl)piperazine-1-carboxylate (200 mg, 0.2 mmol) was dissolved in dichloromethane (5 mL). Subsequently, trimethyl

iodosilane (400 mg, 2 mmol) was added to the above solution under an ice water bath. The reaction solution was continued to be stirred at room temperature for 2 hours. The reaction was quenched with ice water under cooling of the ice bath, and after adjusting the pH to 7-8 with saturated sodium bicarbonate solution, the resultant was extracted with dichloromethane (20 mL x 3 times). The organic phase was washed with saturated brine (20 mL), then dried with anhydrous sodium sulfate, filtered, and finally concentrated under reduced pressure. The resulting residue was purified by preparative high performance liquid chromatography to obtain 5.6 mg of 2-(6-oxo-5-(trifluoromethyl)-1,6-dihydropyridin-3-yl) ethyl-4-(5-(trifluoromethyl)pyrimidin-2-yl) piperazine-1-carboxylate (compound of formula I).

MS (ESI) M/Z: 466.4 $[M+H]^+$.

$^1$H NMR (400 MHz, DMSO-$d_6$) δ 12.21 (s, 1H), 8.73 (s, 2H), 7.90 (d, $J$ = 2.0 Hz, 1H), 7.59 (d, $J$ = 1.9 Hz, III), 4.16 (t, $J$ = 6.3 Hz, 2H), 3.86 - 3.76 (m, 4H). 3.48 - 3.41 (m, 4H), 2.73 (t, $J$ = 6.2 Hz, 2H).

**Example 2 Preparation method of the crystal form IV**

700.2 mg of the compound of formula (I) was weighed into a reaction flask and 50 mL of n-heptane was added. The mixture was stirred at 50 °C for 24 hours. The resultant was filtered, and the resulting solid, which was characterized by XRPD, TGA-DSC and DVS, was the crystal form IV and its XRPD spectrum is substantially as shown in FIG. 1.

The TGA-DSC spectrum is as shown in FIG. 2, and there is no significant weight loss in TGA, indicating that crystal form IV was anhydrous; and the DSC results indicated that the crystal form IV had an endothermic peak at 175 $\pm$ 2 °C.

The DVS spectrum is shown in FIG. 3, and the weight gain of the crystal form IV is 1.07% at 80% RH humidity, indicating a slight hygroscopicity. The comparison of XRPD before and after the DVS test is shown in FIG. 4, and the results show that there is no transformation of the crystal before and after the DVS test.

**Example 3 Preparation method of the crystal form IV**

50.0 mg of the compound of formula (I) was weighed into a vial, 0.2 mL of 2-methyltetrahydrofuran was added to dissolve it. The mixture was filtered into a new vial, 2 mL of n-heptane was slowly dripped therein, and filtered when the solid was precipitated, and the resulting solid, which was characterized by XRPD, TGA-DSC, was the crystal form IV, and its XRPD spectrum is substantially as shown in FIG. 1.

**Example 4 Preparation method of the crystal form VI**

10.6 mg of the compound of formula (I) was weighed into a vial, 1.5 mL of acetone and 2.0 mL of water were added, and the mixture was warmed up to 50 °C to dissolve. The mixture was filtered into a new vial and then slowly cooled down to 0 °C and precipitated to obtain a solid. The resulting solid, which was characterized by XRPD and TGA-DSC, was the crystal form VI, and its XRPD spectrum is substantially as shown in FIG. 5.

The TGA-DSC spectrum is shown in FIG. 6. There was no significant weight loss in TGA, an exothermic peak existed at 105 $\pm$ 2 °C, and an endothermic peak appeared when heating was continued to 175 $\pm$ 2 °C, indicating that the crystal form VI was anhydrous.

**Example 5 Preparation method of the crystal form VI**

9.5 mg of the compound of formula (I) was weighed into a reaction flask, 0.5 mL of acetonitrile and 1.0 mL of water were added. The mixture was stirred at room temperature for 5 days. The resultant was filtered, and the resulting solid, which was characterized by XRPD, was the crystal form VI, and its XRPD spectrum was substantially as shown in FIG. 5.

**Example 6 Preparation method of the crystal form VI**

10.3 mg of the compound of formula (I) was weighed into a vial, 1.5 mL of acetone and 2.0 mL of water were added to dissolve it at 35 °C. The mixture was filtered and slowly cooled down to 5 °C and stirred at a maintained temperature for 1 hour. The resultant was filtered and dried under vacuum at 50 °C for 4 hours. The resulting solid, which was characterized by XRPD and TGA-DSC, was the crystal form VI. The XRPD result is substantially as shown in FIG. 5.

The TGA-DSC spectrum is shown in FIG. 6. There is a 1.56% weight loss between 35-117 °C in TGA, but there is no corresponding endothermic peak, which is a surface solvent desorption process, indicating that the crystal form VI is anhydrous; the DSC results indicate that the crystal form has an exothermic peak at 105 $\pm$ 2 °C, and an adsorption peak at 177 $\pm$ 2 °C in its TGA-DSC profile.

### Example 7 Preparation method of the crystal form VIII

10.7 mg of the compound of formula (I) was weighed, 1.0 mL of dichloromethane was added to dissolve it. The mixture was filtered into a vial, then 4.0 mL of n-heptane was slowly dripped, and a solid was precipitated. After filtration, XRPD and TGA-DSC characterization was carried out, and the solid was the crystal form VIII, and its XRPD spectrum was substantially as shown in FIG. 7.

The TGA-DSC spectrum is shown in FIG. 8. There is no significant weight loss in its TGA, and the DSC profile has an endothermic peak at 124 ± 2 °C and 179 ± 2 °C, and an exothermic peak at 128 ± 2 °C.

### Example 8 Preparation method of the crystal form X

10.2 mg of the crystal form I of the compound of formula (I) was weighed, 1.0 mL of ethanol was added and the mixture was pulped at room temperature for 5 days, and the solid was obtained by filtration, which was characterized by XRPD, TGA-DSC and DVS, was the crystal form X, and its XRPD spectrum was substantially as shown in FIG. 9.

The TGA-DSC spectrum was substantially as shown in FIG. 10, and there was no significant weight loss in TGA, indicating that the crystal form X was anhydrous; the DSC results showed that the crystal form X had an endothermic peak at 179 ± 2 °C.

The DVS spectrum is shown in FIG. 11, and the weight gain is 1.4% at 80% RH humidity, indicating a slight hygroscopicity. The comparison of XRPD before and after the DVS test is shown in FIG. 12. There is no transformation of the crystal before and after the DVS test.

### Example 9 Preparation method of the sodium salt of the compound of formula (I-1)

1.0 g of the compound of formula (I) was weighed, 1.8 mL of anhydrous ethanol was added to dissolve it at 70 °C. The mixture was filtered; 1.05 eq of sodium ethoxide solution was added dropwise to form a salt, and the mixture was reacted for 30 min; filtrated by suction, and dried at 50 °C for 2 hours to obtain the sodium salt of the compound of formula (I-1).

### Example 10 Preparation method of the crystal form I

10.5 mg of the compound of formula (I-1) was weighed into a reaction flask, 0.1 mL of tetrahydrofuran was added to dissolve it with stirring at room temperature. The mixture was filtered, then 0.4 mL of n-heptane was added slowly dropwise and a solid was precipitated, centrifuged and dried. The resulting solid, which was characterized by XRPD, TGA-DSC and DVS, was the crystal form I, and its XRPD spectrum is substantially as shown in FIG. 13.

The TGA-DSC spectrum is shown in FIG. 14, and there was no significant weight loss in TGA, indicating that the crystal form I was anhydrous; the DSC results indicated that the crystal form I had an exothermic peak at 241 ± 2 °C.

The DVS spectrum is shown in FIG. 15, the crystal form I gained weight by 13.29% at 80% RH humidity, and had hygroscopicity.

### Example 11 Preparation method of the crystal form I

10.3 mg of the compound of formula (I) was weighed into a vial, 0.1 mL of tetrahydrofuran was added to dissolve it. The mixture was filtered into a new vial, and 0.4 mL of methyl isobutyl ketone was dropped slowly. Filtration was performed when the solid was precipitated. The resulting solid, which was characterized by XRPD and TGA-DSC, was the crystal form I, and its XRPD spectrum was substantially as shown in FIG. 13.

### Example 12 Influence factor tests

The stability of the crystal form IV of compounds of formula (I) was investigated at different temperatures as well as humidity with reference to Guidelines for Stability Test of Bulk Drugs and Formulations, Chinese Pharmacopoeia 2020 Edition. The purity was assayed using HPLC and the crystal form was assayed using XRPD on day 0, 2 and 7, and the experimental results are shown in Table 11.

Table 11 Influencing factor tests for the crystal form IV

| Sample | Process | Time | Purity (Area %) | XRPD |
|---|---|---|---|---|
| | T0 | day 0 | 99.73 | Crystal form IV |

(continued)

| Sample | Process | Time | Purity (Area %) | XRPD |
|---|---|---|---|---|
| Crystal form IV of the compound of formula (I) | 60 °C | day 2 | 99.73 | Crystal form IV |
| | | day 7 | 99.65 | Crystal form IV |
| | 40 °C/75% RH | day 2 | 99.72 | Crystal form IV |
| | | day 7 | 99.62 | Crystal form IV |
| Conclusion: The crystal form IV is relatively stable under high temperature and high humidity conditions in terms of physicochemical properties. | | | | |

The stability of the crystal form I of sodium salt of compounds of formula (I) was investigated at different temperatures as well as humidity with reference to Guidelines for Stability Test of Bulk Drugs and Formulations, Chinese Pharmacopoeia 2020 Edition. The purity was assayed using HPLC and the crystal form was assayed using XRPD on day 0, 5 and 10, and the experimental results are shown in Table 12.

Table 12 Influencing factors test for the crystal form I

| Sample | Process | Time | Purity (Area %) | XRPD |
|---|---|---|---|---|
| Crystal form I | T0 | day 0 | 99.69% | Crystal form I of the sodium salt |
| | 40 °C | day 5 | 99.71 % | Crystal form I of the sodium salt |
| | | day 10 | 99.70% | Crystal form I of the sodium salt |
| | 60 °C | day 5 | 99.72% | Crystal form I of the sodium salt |
| | | day 10 | 99.67% | Crystal form I of the sodium salt |
| | 25 °C/75% RH | day 5 | 99.69% | Crystal transformation |
| | | day 10 | 99.72% | Crystal transformation |
| | 25 °C/90% RH | day 5 | 99.71 % | Crystal transformation |
| | | day 10 | 99.74% | Crystal transformation |
| | Light exposure | day 5 | 99.67% | Crystal form I |
| | | day 10 | 99.61 % | Crystal form I of the sodium salt |
| Conclusion: The crystal form I is physicochemical stable at high temperature, and a transformation of the crystal occurs under high humidity conditions. | | | | |

### Example 13 Hygroscopicity test

The moisture adsorption/desorption data of the crystal form IV of the compound of formula (I) was tested with reference to Guidelines for Hygroscopicity Test of Drugs, Chinese Pharmacopoeia 2020 Edition. The DVS curve of the crystal form IV is shown in FIG. 3, and the hygroscopicity data thereof is shown in Table 13. The XRPD of the remaining solids after testing the DVS experiment was unchanged for the crystal form IV, as shown in FIG. 4.

Table 13 Hygroscopicity of the crystal form IV

| Sample | Weight gain at 80% RH | Weight gain at 90% RH | Hygroscopicity evaluation | Crystal form after DVS |
|---|---|---|---|---|
| Crystal form IV | 1.07% | 1.11% | Slightly hygroscopic | Crystal form IV |
| Conclusion: From the hygroscopicity test, it can be seen that the crystal form IV is slightly hygroscopic. | | | | |

The moisture adsorption/desorption data of the crystal form I of the sodium salt of the compound of formula (I) were tested with reference to Guidelines for Hygroscopicity Test of Drugs, Chinese Pharmacopoeia 2020 Edition. The DVS curve of the crystal form I is as shown in the figures, the hygroscopicity data thereof is shown in Table 14.

Table 14 Hygroscopicity of the crystal form I

| Sample | Weight gain at 80% RH | Weight gain at 90% RH | Hygroscopicity evaluation |
|---|---|---|---|
| Crystal form I | 13.29% | 13.77% | Hygroscopic |
| Conclusion: From the hygroscopicity test, it can be seen that the crystal form I is slightly hygroscopic. | | | |

**Biological test evaluation:**

**I. PARP7 in vitro enzymatic assay**

In this experiment, the inhibitory effect of the compound of formula (I) on PARP7 enzyme activity was tested by serial dilution of the tested compounds and assayed in replicates.

1. A histone-coated 384-well plate was prepared by adding 25 μL of histone solution to each well and incubating at 4 °C overnight.

2. PBST buffer, blocking buffer and assay buffer were prepared.

3. The histone-coated 384-well plate was washed 3 times using PBST buffer. Blocking was performed at room temperature for 1 hour using 50 μL of blocking buffer. The plate was washed 3 times using PBST buffer.

4. Compound preparation:

   A 2000× compound was prepared in a 96-well source plate. 50 nL of the compound was transferred from the source plate to a 96-well intermediate plate. Each well was supplemented with 39.95 μL of assay buffer. The plate was shaken well and centrifuged at 1000 rpm for 1 minute.

   5 μL of the compound in DMSO was transferred to each well.

5. Enzymatic reaction:

   An enzyme mixture was incubated at 25 °C for 10 minutes.

   10 μL of the enzyme mixture was added and incubated with the compound for 10 minutes at room temperature.

   10 μL of assay buffer was added to the negative control wells of the assay plate.

   10 μL of 2.5× Biotin-NAD+ was added to each well, followed by incubation at 25 °C for 60 min.

   The plate was washed 3 times using PBST buffer.

6.Assay:

   25 μL of Stre-HRP was added.

   The plate was incubated at room temperature for 1 hour and washed 3 times using PBS buffer.

   25 μL of QuantaRed Enhancer Mix was added and incubated for 10 minutes.

   2.5 μL of QuantaRed Stop Solution was added to stop the peroxidase reaction, and the plate was shaken for 10-30 seconds.

7. The plate was immediately read using Paradigm and detected for reads at Ex550/Em620.

8. Data processing

   The data were fitted using Equation (1) in Excel to obtain the inhibition values;

suppression % = (max signal value - target signal value)/(max signal value - min signal value) $\times$ 100%; Equation (1):

The data in XL-Fit were fitted using Equation (2) to obtain the $IC_{50}$ value;

$$\text{Equation (2): } Y=Bottom+(Top-Bottom)/(1+(IC_{50}/X)\times HillSlope);$$

Y is the percentage of inhibition, and X is the compound concentration.

**Experimental result**

Table 15: The result of PARP7 enzymatic inhibition

| Compound | PARP7 $IC_{50}$ (nM) |
|---|---|
| Compound of formula (I) | 1.8 |
| Conclusion: The compound of formula (I) can effectively inhibit PARP7 enzyme activity. | |

**II. In vivo pharmacokinetic determination in mice and rats**

1.Experimental purpose:

To study the in vivo plasma pharmacokinetic behavior of the compounds of the present application after single intravenous bolus injection and oral administration, using male C57BL/6 mice or SD male rats as tested animals.

2.Test protocol

2.1 Test animals

Healthy adult C57BL/6 mice or SD rats (3 rats/group), male, from Shanghai Jihui Laboratory Animal Breeding Co., Ltd. and Charles River Laboratories Co., Ltd.

2.2 Drug administration

The intravenous bolus injection and oral groups were consisted of 3 mice or rats, and the dose of intravenous bolus injection was 1 mg/kg in a volume of 5 mL/kg, and the dose of oral administration was 5 mg/kg in a volume of 10 mL/kg. The solvent was 5% DMSO/5% Kolliphor HS15/90% Saline.

The structure of the control compound RBN-2397 is shown below:

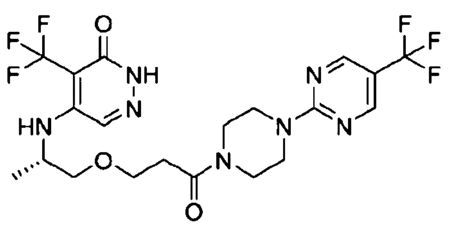

2.3 Experimental equipment

The centrifuge was purchased from Eppendorf and the pipette was purchased from Eppendorf.

2.4 Sample collection

After the animals were administered, 0.02 mL of blood was collected intravenously at 0.0833(IV), 0.25, 0.5, 1, 2, 4, 8, and 24 hours. Blood was placed in EDTA-K2 tubes, centrifuged at 4 °C and 4600 rpm for 5 min to separate the plasma, and stored at -80 °C.

2.5 Sample processing

1) 10 μL of plasma sample was precipitated by adding 200 μL of acetonitrile, vortexed and mixed, and centrifuged for 15 min.
2) The supernatant after treatment was diluted with water, and then analyzed for the concentration of the tested compounds by LC/MS/MS.

2.6 Bioanalysis

Liquid phase conditions: Shimadzu LC-30AD;
Mass spectrometry conditions: AB Sciex API 5500;
Chromatographic column: Phenomenex Kinetex 2.6 μm C18;
Mobile phase: A: 5 mM ammonium acetate aqueous solution (containing 0.05 vol% of formic acid); B: acetonitrile (containing 0.1 vol% of formic acid) flow rate: 0.5 mL/min;
The elution gradient is shown in Table 16:

Table 16

| Time (min) | A (%) | B (%) |
|---|---|---|
| 0.00 | 85.0 | 10.0 |
| 0.50 | 85.0 | 10.0 |
| 2.00 | 5.00 | 95.0 |
| 2.20 | 5.00 | 95.0 |
| 2.21 | 85.0 | 10.0 |
| 2.50 | 85.0 | 10.0 |

**Experimental results and analysis**

Pharmacokinetic parameters were calculated using WinNonlin 8.0 and the pharmacokinetic parameters of the drugs administered intravenously and orally to mice or rats are shown in Tables 17 and 18 below. Wherein, "Dose" denotes the administered dose, "CL" denotes the plasma clearance, "$V_{ss}$" denotes the volume of distribution at steady state of the drug, "$T_{1/2}$" denotes the half-life of the drug, "AUC" denotes the area under the concentration-time curve, "$C_{max}$" denotes the peak drug concentration, and "F" indicates bioavailability.

Table 17 Pharmacokinetic parameters of the compounds of the present application administered intravenously to mice or rats

| Compound | Animals | Dose (mg/kg) | CL (L/hr/kg) | $V_{ss}$ (L/kg) | $T_{1/2}$ (hr) | AUC (hr*ng/mL) |
|---|---|---|---|---|---|---|
| RBN-2397 | C57BL/6 mice | 1 | 3.4 | 0.6 | 0.2 | 288 |
| | SD rats | 1 | 5.1 | 1.2 | 0.2 | 193 |
| Compound of formula (I) | C57BL/6 mice | 1 | 0.09 | 0.6 | 6.1 | 10408 |
| | SD rats | 1 | 0.2 | 0.9 | 4.7 | 5745 |

Table 18. Pharmacokinetic parameters of the compounds of the present application administered orally to mice or rats

| Compound | Animals | Dose (mg/kg) | $C_{max}$ (ng/mL) | AUC (hr*ng/mL) | F (%) |
|---|---|---|---|---|---|
| RBN-2397 | C57BL/6 mice | 5 | 612 | 203 | 14 |
| | SD rats | 5 | 319 | 264 | 27 |
| Compound of formula (I) | C57BL/6 mice | 5 | 9313 | 42155 | 80 |
| | SD rats | 5 | 2350 | 13798 | 47 |
| Conclusion: at the same dose, the compound of formula (I) of the present application showed significantly higher exposure in mouse and rat plasma after intravenous bolus injection and oral administration than the control compound RBN-2397. | | | | | |

The above is only a preferred embodiment of the present application and is not intended to limit the present application, and any modifications, equivalent substitutions, improvements, etc. made within the spirit and principles of the present application shall be included within the claimed scope of the present application.

## Claims

1. A crystal form IV of a compound of formula (I),

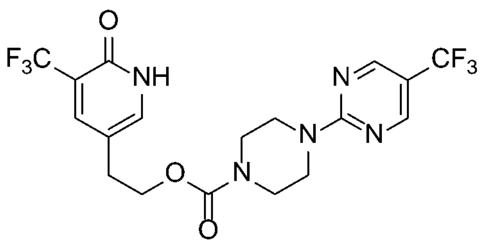

(I)

wherein an X-ray powder diffraction pattern of the crystal form IV has characteristic peaks at 2θ values of 14.13°, 15.56°, 17.33°, 18.07°, and 22.30°, each with a 2θ error range of ± 0.2°, as measured using Cu-$K_\alpha$ radiation.

2. The crystal form IV according to claim 1, wherein the X-ray powder diffraction pattern of the crystal form IV has characteristic peaks at 2θ values of 14.13°, 14.46°, 15.56°, 17.02°, 17.33°, 18.07°, 19.17°, and 22.30°, each with a 2θ error range of ± 0.2°, as measured using Cu-$K_\alpha$ radiation.

3. The crystal form IV according to claim 2, wherein the X-ray powder diffraction pattern of the crystal form IV has characteristic peaks at 2θ values of 7.25°, 10.00°, 14.13°, 14.46°, 15.56°, 17.02°, 17.33°, 18.07°, 19.17°, and 22.30°, each with a 2θ error range of ± 0.2°, as measured using Cu-$K_\alpha$ radiation.

4. The crystal form IV according to claim 3, wherein the X-ray powder diffraction pattern of the crystal form IV is substantially as shown in FIG. 1.

5. The crystal form IV according to any one of claims 1-4, wherein a TGA-DSC profile of the crystal form IV has an endothermic peak at 175 ± 2 °C.

6. The crystal form IV according to any one of claims 1-5, wherein the TGA-DSC profile of the crystal form IV is substantially as shown in FIG. 2.

7. A crystal form VI of a compound of formula (I),

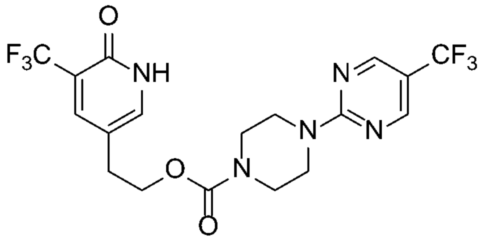

(I)

wherein an X-ray powder diffraction pattern of the crystal form VI has characteristic peaks at 2θ values of 5.20°, 7.49°, 10.70°, 13.42°, 14.30°, 14.85°, 16.01°, and 18.66°, each with a 2θ error range of ± 0.2°, as measured using Cu-$K_\alpha$ radiation.

**8.** The crystal form VI according to claim 7, wherein the X-ray powder diffraction pattern of the crystal form VI has characteristic peaks at 2θ values of 5.20°, 7.49°, 7.95°, 9.70°, 10.70°, 11.53°, 13.05°, 13.42°, 14.30°, 14.85°, 16.01°, 16.38°, 17.11°, and 18.66°, each with a 2θ error range of ± 0.2°, as measured using Cu-$K_\alpha$ radiation.

**9.** The crystal form VI according to claim 8, wherein the X-ray powder diffraction pattern of the crystal form VI has characteristic peaks at 2θ values of 5.20°, 6.68°, 7.49°, 7.95°, 9.70°, 10.70°, 11.53°, 13.05°, 13.42°, 13.67°, 14.30°, 14.85°, 16.01°, 16.38°, 17.11°, 18.66°, 19.37°, 23.44°, and 24.36°, each with a 2θ error range of ± 0.2°, as measured using Cu-$K_\alpha$ radiation.

**10.** The crystal form VI according to claim 9, wherein the X-ray powder diffraction pattern of the crystal form VI is substantially as shown in FIG. 5.

**11.** The crystal form VI according to any one of claims 7-10, wherein a TGA-DSC profile of the crystal form VI has an exothermic peak at 105 ± 2 °C and an endothermic peak at 177 ± 2 °C.

**12.** The crystal form VI according to any one of claims 7-11, wherein the TGA-DSC profile of the crystal form VI is substantially as shown in FIG. 6.

**13.** A crystal form VIII of a compound of formula (I),

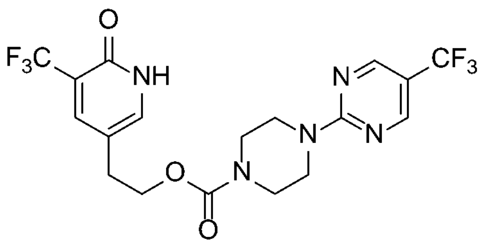

(I)

wherein an X-ray powder diffraction pattern of the crystal form VIII has characteristic peaks at 2θ values of 8.97°, 13.51°, 16.20°, 18.05°, 18.56°, 20.94°, and 21.25°, each with a 2θ error range of ± 0.2°, as measured using Cu-$K_\alpha$ radiation.

**14.** The crystal form VIII according to claim 13, wherein the X-ray powder diffraction pattern of the crystal form VIII has characteristic peaks at 2θ values of 8.97°, 13.51°, 15.51°, 16.20°, 18.05°, 18.56°, 19.94°, 20.94°, 21.25°, and 27.13°, each with a 2θ error range of ± 0.2°, as measured using Cu-$K_\alpha$ radiation.

**15.** The crystal form VIII according to claim 14, wherein the X-ray powder diffraction pattern of the crystal form VIII has characteristic peaks at 2θ values of 8.97°, 10.75°, 13.51°, 15.51°, 16.20°, 17.88°, 18.05°, 18.56°, 19.94°, 20.94°, 21.25°, 22.67°, 25.07°, and 27.13°, each with a 2θ error range of ± 0.2°, as measured using Cu-$K_\alpha$ radiation.

**16.** The crystal form VIII according to claim 15, wherein the X-ray powder diffraction pattern of the crystal form VIII is substantially as shown in FIG. 7.

**17.** The crystal form VIII according to any one of claims 13-16, wherein a TGA-DSC profile of the crystal form VIII has endothermic peaks at 124 ± 2 °C and 179 ± 2 °C and an exothermic peak at 128 ± 2 °C.

**18.** The crystal form VIII according to any one of claims 13-17, wherein the TGA-DSC profile of the crystal form VIII is substantially as shown in FIG. 8.

**19.** A crystal form X of a compound of formula (I),

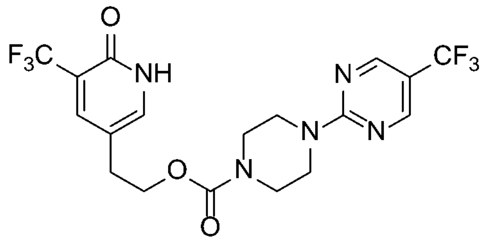

(I)

wherein an X-ray powder diffraction pattern of the crystal form X has characteristic peaks at 2θ values of 11.61°, 15.66°, 17.49°, 18.36°, 19.51°, 21.28°, 23.60°, and 25.43°, each with a 2θ error range of ± 0.2°, as measured using Cu-$K_\alpha$ radiation.

**20.** The crystal form X according to claim 19, wherein the X-ray powder diffraction pattern of the crystal form X has characteristic peaks at 2θ values of 11.61°, 14.07°, 14.70°, 15.66°, 17.49°, 18.36°, 18.54°, 19.51°, 21.28°, 23.60°, and 25.43°, each with a 2θ error range of ± 0.2°, as measured using Cu-$K_\alpha$ radiation.

**21.** The crystal form X according to claim 20, wherein the X-ray powder diffraction pattern of the crystal form X has characteristic peaks at 2θ values of 11.24°, 11.61°, 14.07°, 14.70°, 14.88°, 15.66°, 15.84°, 17.49°, 18.36°, 18.54°, 19.51°, 19.90°, 21.28°, 23.60°, 25.43°, and 27.67°, each with a 2θ error range of ± 0.2°, as measured using Cu-$K_\alpha$ radiation.

**22.** The crystal form X according to claim 21, wherein the X-ray powder diffraction pattern of the crystal form X is substantially as shown in FIG. 9.

**23.** The crystal form X according to any one of claims 19-22, wherein a TGA-DSC profile of the crystal form X has an endothermic peak at 179 ± 2 °C.

**24.** The crystal form X according to any one of claims 19-23, wherein the TGA-DSC profile of the crystal form X is substantially as shown in FIG. 10.

**25.** A pharmaceutically acceptable salt of a compound of formula (I),

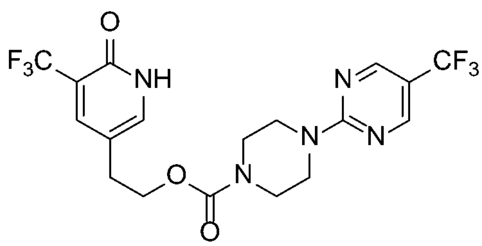

(I).

**26.** The pharmaceutically acceptable salt of the compound of formula (I) according to claim 25, wherein it has a following structure:

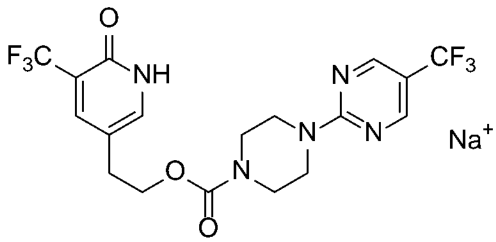

(I-1).

**27.** A crystal form I of the pharmaceutically acceptable salt of the compound of formula (I) according to claim 25 or 26, wherein an X-ray powder diffraction pattern of the crystal form I has characteristic peaks at 2θ values of 15.99°, 17.16°, 17.29°, 17.70°, and 23.24°, each with a 2θ error range of ± 0.2°, as measured using Cu-$K_\alpha$ radiation.

**28.** The crystal form I according to claim 27, wherein the X-ray powder diffraction pattern of the crystal form I has characteristic peaks at 2θ values of 8.99°, 15.99°, 17.16°, 17.29°, 17.70°, 21.39°, 22.92°, and 23.24°, each with a 2θ error range of ± 0.2°, as measured using Cu-$K_\alpha$ radiation.

**29.** The crystal form I according to claim 28, wherein the X-ray powder diffraction pattern of the crystal form I has characteristic peaks at 2θ values of 8.99°, 12.92°, 15.99°, 17.16°, 17.29°, 17.70°, 20.98°, 21.39°, 22.92°, and 23.24°, each with a 2θ error range of ± 0.2°, as measured using Cu-$K_\alpha$ radiation.

**30.** The crystal form I according to claim 29, wherein the X-ray powder diffraction pattern of the crystal form I with Cu-$K_\alpha$ radiation is substantially as shown in FIG. 13.

**31.** The crystal form I according to any one of claims 27-30, wherein a TGA-DSC profile of the crystal form I has an exothermic peak at 241 ± 2 °C.

**32.** The crystal form I according to any one of claims 27-31, wherein the TGA-DSC profile of the crystal form I is substantially as shown in FIG. 14.

**33.** A pharmaceutical composition comprising the crystal form IV according to any one of claims 1-6, the crystal form VI according to any one of claims 7-12, the crystal form VIII according to any one of claims 13-18, the crystal form X according to any one of claims 19-24, the pharmaceutically acceptable salt of the compound of formula (I) according to claim 25 or 26, or the crystal form I according to any one of claims 27-32, and one or more pharmaceutically acceptable carrier(s).

**34.** Use of the crystal form IV according to any one of claims 1-6, the crystal form VI according to any one of claims 7-12, the crystal form VIII according to any one of claims 13-18, the crystal form X according to any one of claims 19-24, the pharmaceutically acceptable salt of the compound of formula (I) according to claim 25 or 26, the crystal form I according to any one of claims 27-32, or the pharmaceutical composition according to claim 33 in the preparation of a medicament for treating and/or preventing a tumor.

**35.** The use according to claim 34, wherein a formation of the tumor is associated with PARP; preferably, the PARP is PARP7.

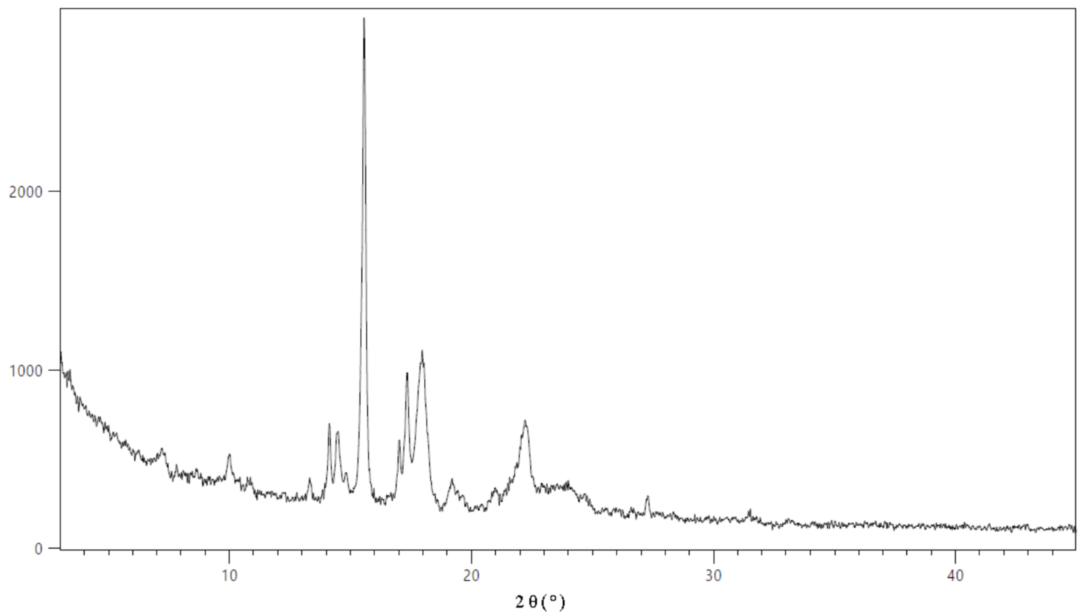
2000
1000
0
10
20
30
40
2θ(°)

FIG. 1

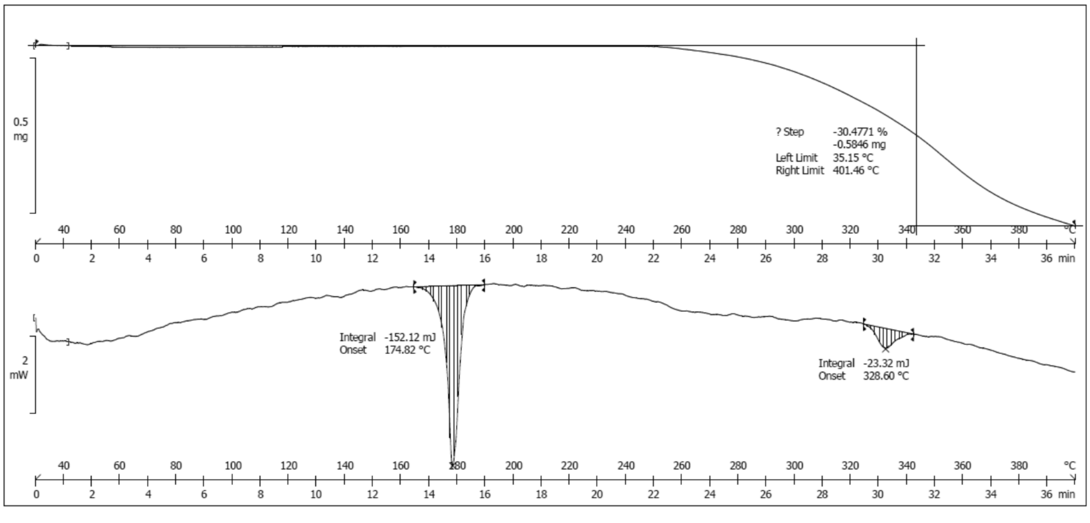
0.5
mg
? Step -30.4771 %
-0.5846 mg
Left Limit 35.15 °C
Right Limit 401.46 °C
2
mW
Integral -152.12 mJ
Onset 174.82 °C
Integral -23.32 mJ
Onset 328.60 °C
°C
min

FIG. 2

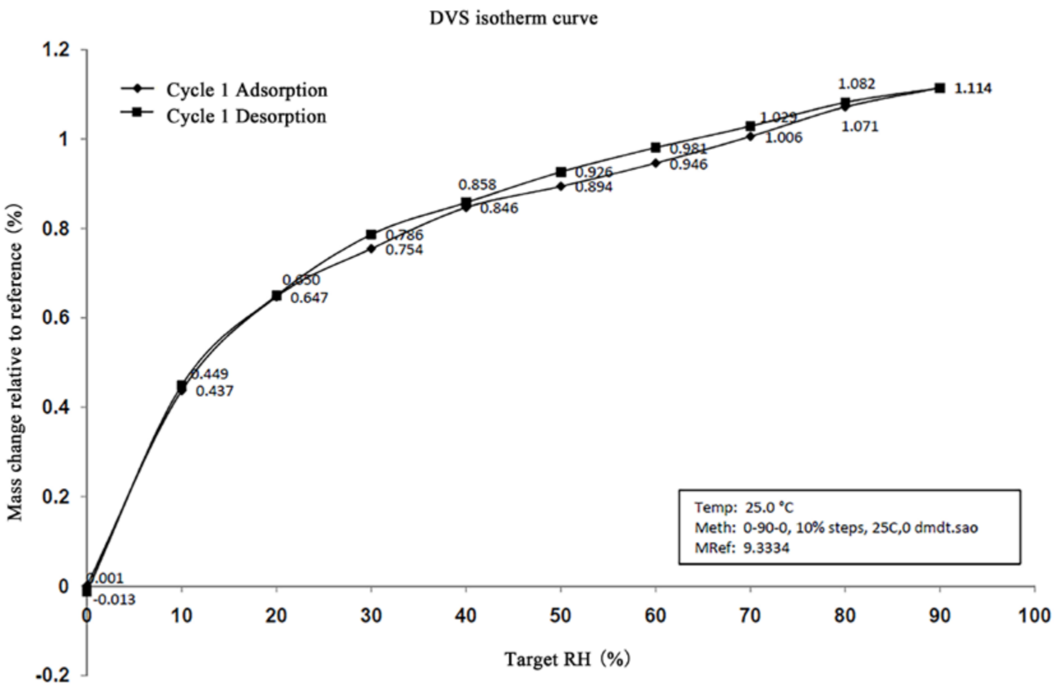
DVS isotherm curve
Cycle 1 Adsorption
Cycle 1 Desorption
Mass change relative to reference (%)
Target RH (%)
Temp: 25.0 °C
Meth: 0-90-0, 10% steps, 25C,0 dmdt.sao
MRef: 9.3334

FIG. 3

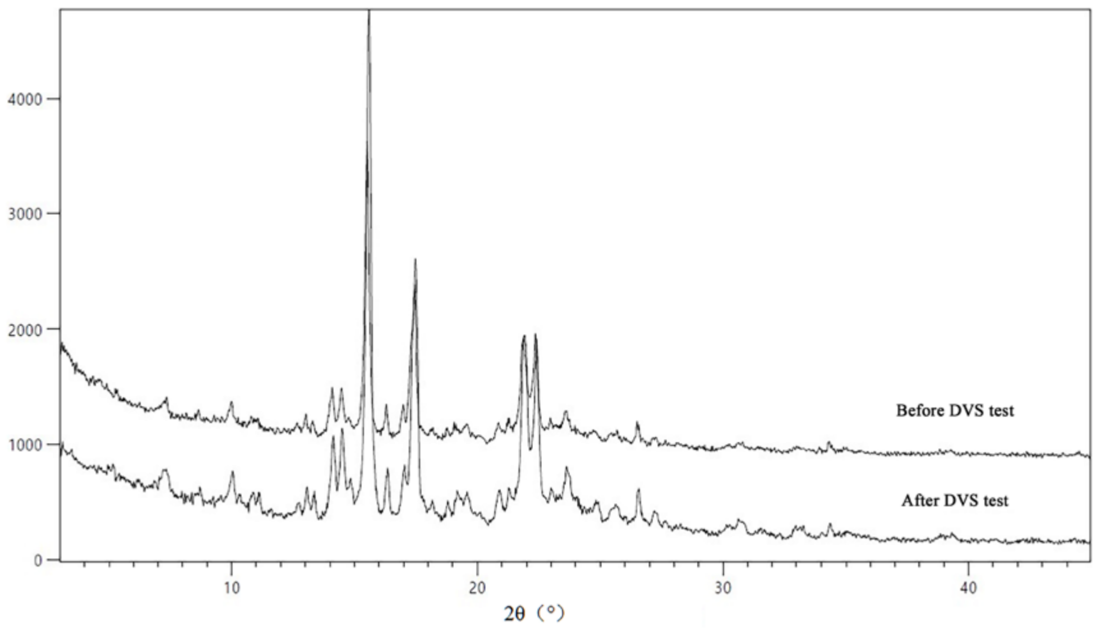
Before DVS test
After DVS test
2θ (°)

FIG. 4

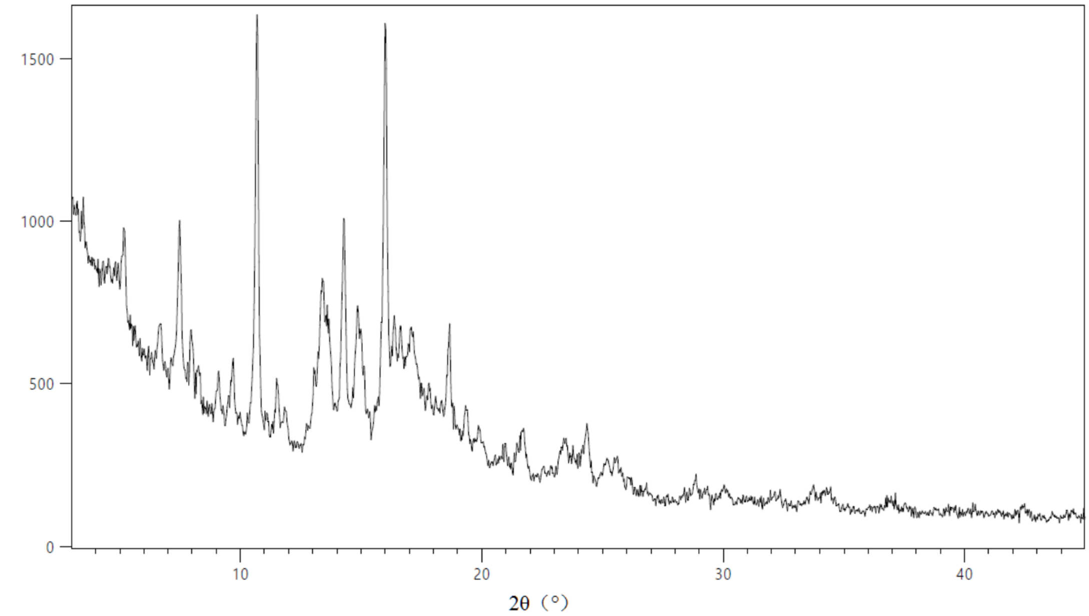
1500
1000
500
0
10
20
30
40
2θ (°)

FIG. 5

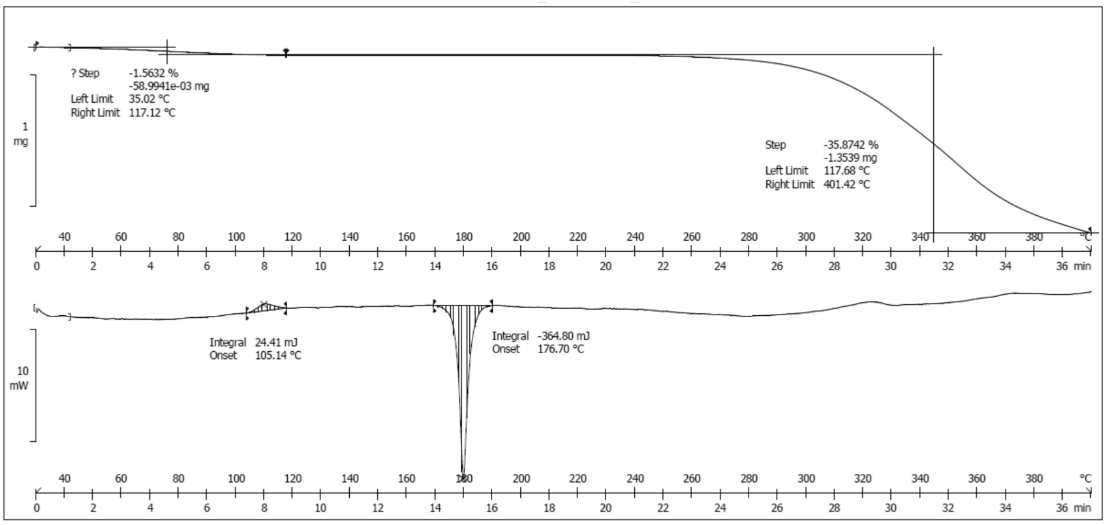
? Step -1.5632 %
-58.9941e-03 mg
Left Limit 35.02 °C
Right Limit 117.12 °C
1 mg
Step -35.8742 %
-1.3539 mg
Left Limit 117.68 °C
Right Limit 401.42 °C
Integral 24.41 mJ
Onset 105.14 °C
Integral -364.80 mJ
Onset 176.70 °C
10 mW
°C
min

FIG. 6

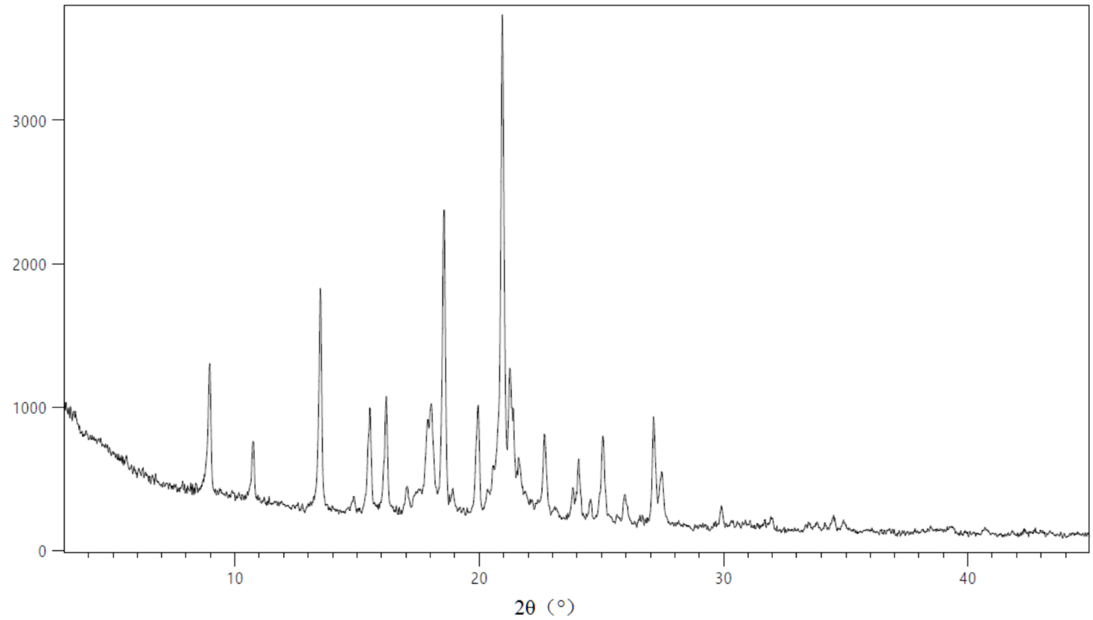
3000
2000
1000
0
10
20
30
40
2θ (°)

FIG. 7

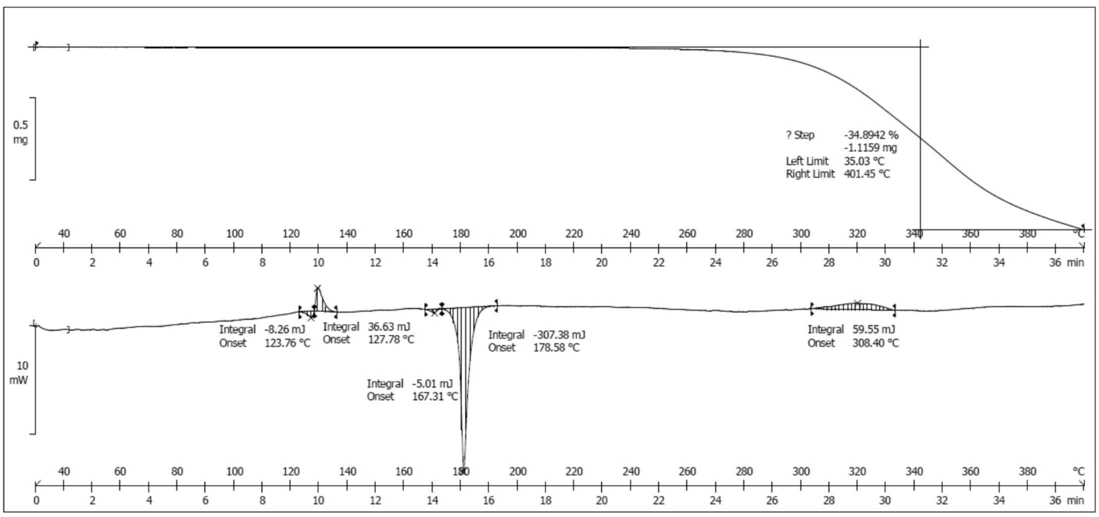
0.5
mg
? Step -34.8942 %
-1.1159 mg
Left Limit 35.03 °C
Right Limit 401.45 °C
10
mW
Integral -8.26 mJ
Onset 123.76 °C
Integral 36.63 mJ
Onset 127.78 °C
Integral -307.38 mJ
Onset 178.58 °C
Integral -5.01 mJ
Onset 167.31 °C
Integral 59.55 mJ
Onset 308.40 °C
°C
min

FIG. 8

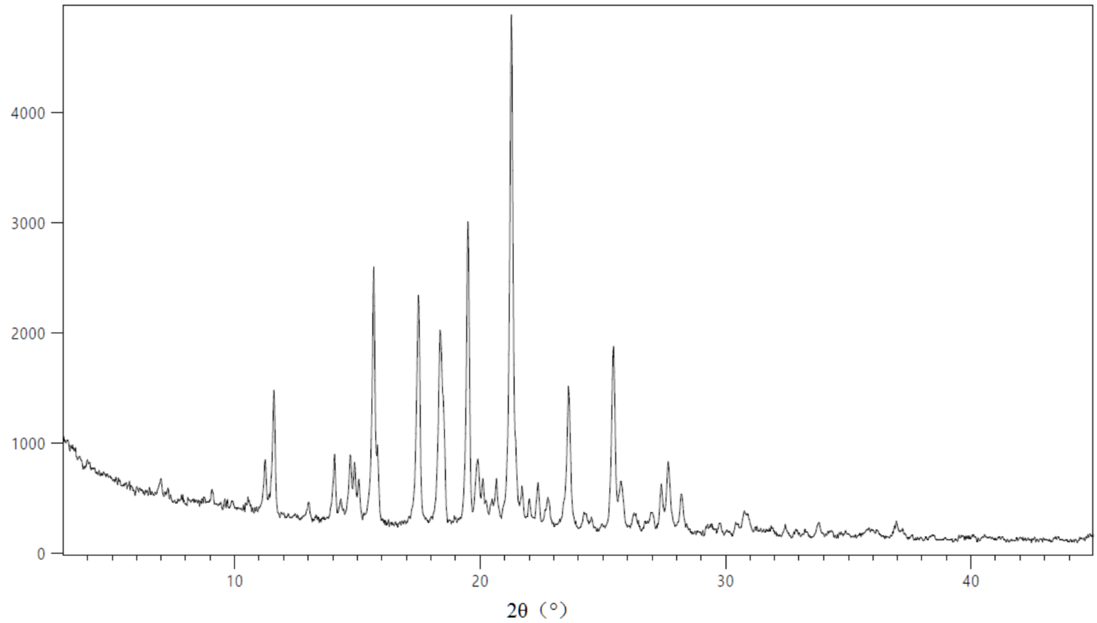
4000
3000
2000
1000
0
10
20
30
40
2θ (°)

FIG. 9

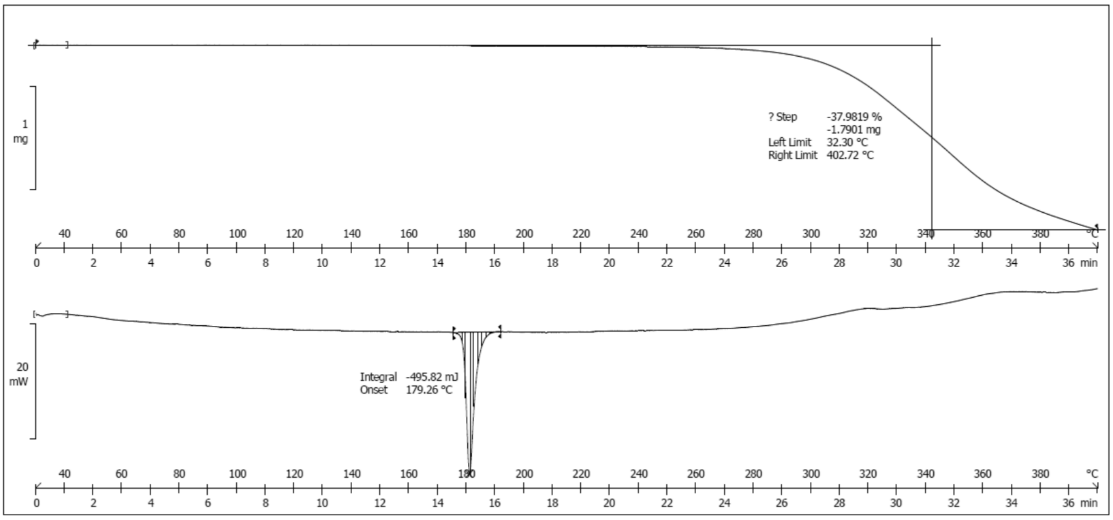
1 mg
? Step -37.9819 %
-1.7901 mg
Left Limit 32.30 °C
Right Limit 402.72 °C
20 mW
Integral -495.82 mJ
Onset 179.26 °C
°C
min

FIG. 10

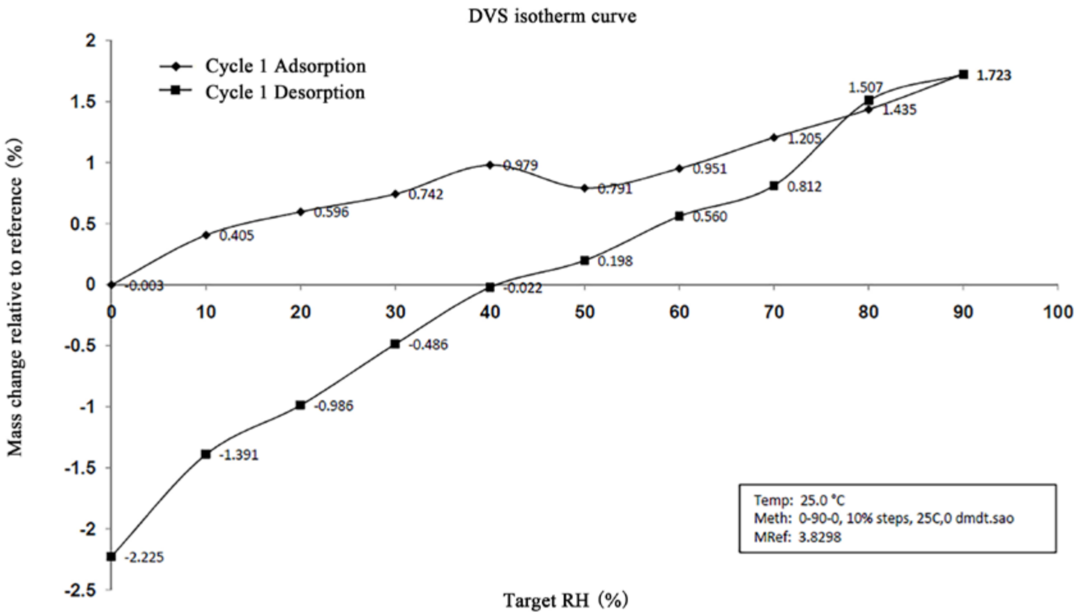
DVS isotherm curve
Cycle 1 Adsorption
Cycle 1 Desorption
Mass change relative to reference (%)
Target RH (%)
Temp: 25.0 °C
Meth: 0-90-0, 10% steps, 25C,0 dmdt.sao
MRef: 3.8298
-0.003
0.405
0.596
0.742
0.979
0.791
0.951
1.205
1.435
1.723
1.507
0.812
0.560
0.198
-0.022
-0.486
-0.986
-1.391
-2.225

FIG. 11

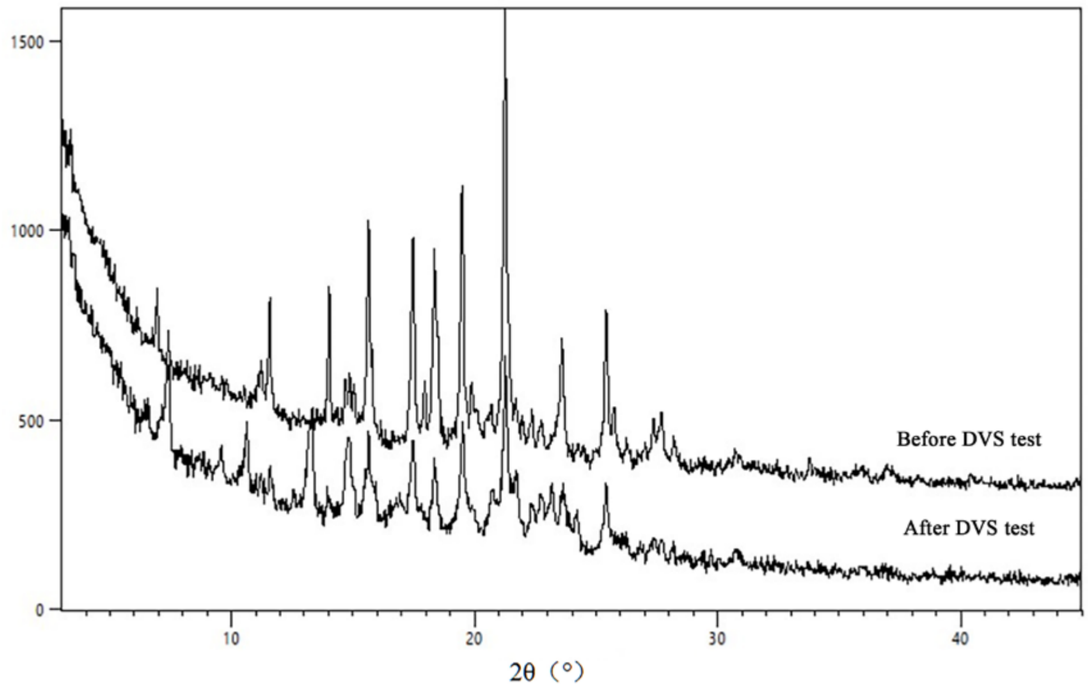
Before DVS test
After DVS test
2θ (°)

FIG. 12

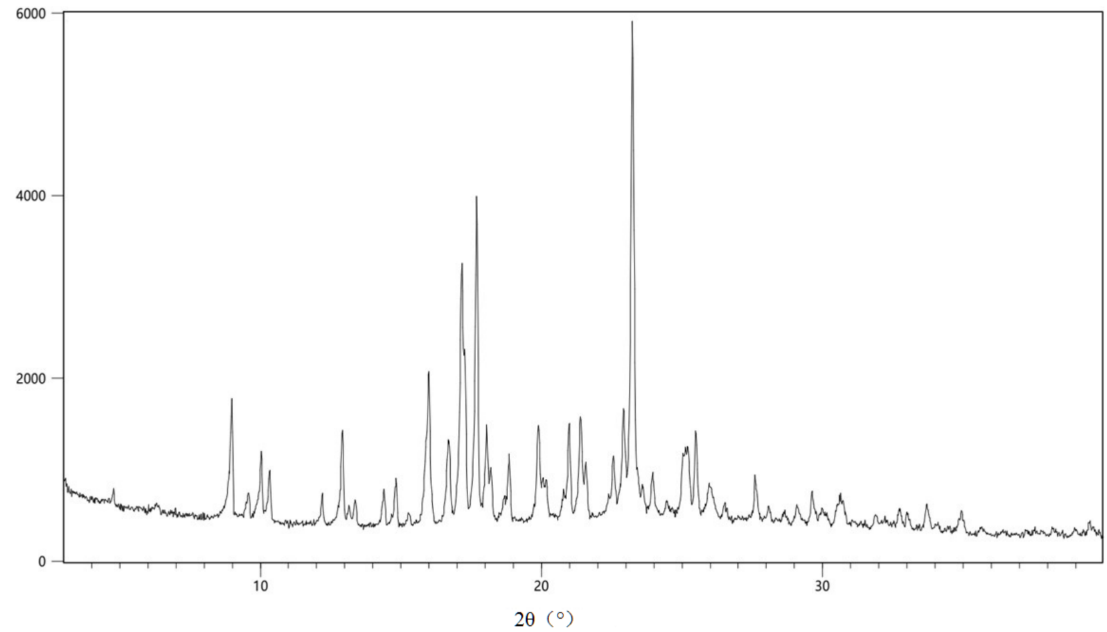
6000
4000
2000
0
10
20
30
2θ (°)

FIG. 13

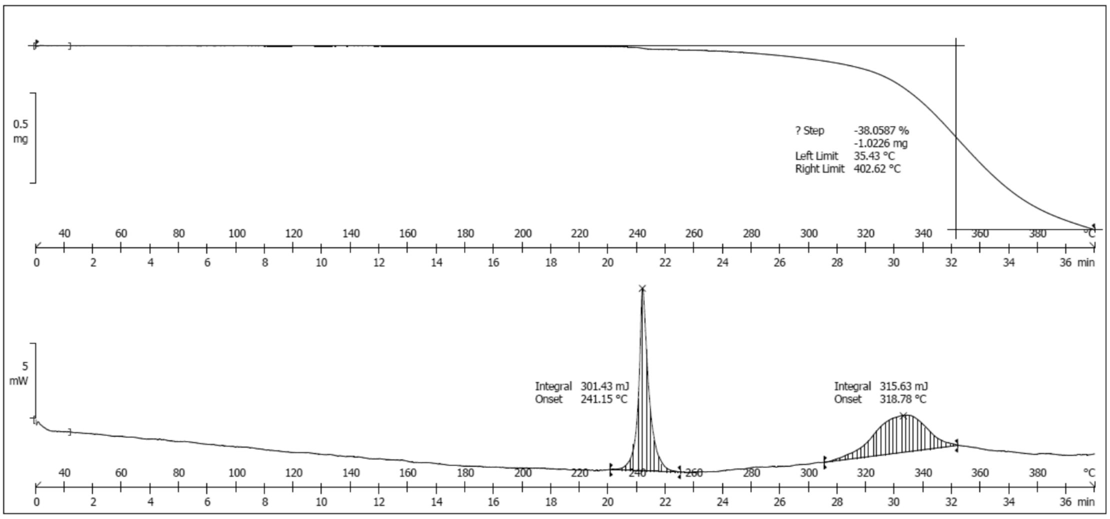
0.5
mg
? Step -38.0587 %
-1.0226 mg
Left Limit 35.43 °C
Right Limit 402.62 °C
5
mW
Integral 301.43 mJ
Onset 241.15 °C
Integral 315.63 mJ
Onset 318.78 °C
°C
min

FIG. 14

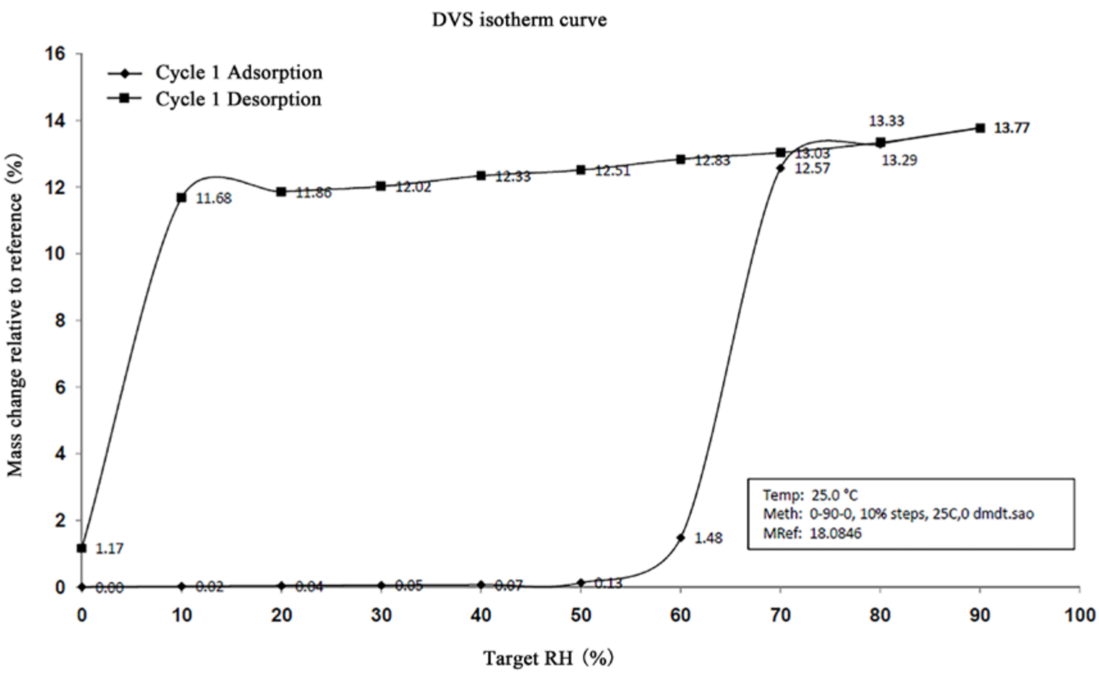
DVS isotherm curve
Cycle 1 Adsorption
Cycle 1 Desorption
Mass change relative to reference (%)
Target RH (%)
0.00
0.02
0.04
0.05
0.07
0.13
1.48
12.57
13.29
13.77
13.33
13.03
12.83
12.51
12.33
12.02
11.86
11.68
1.17
Temp: 25.0 °C
Meth: 0-90-0, 10% steps, 25C,0 dmdt.sao
MRef: 18.0846

FIG. 15

**INTERNATIONAL SEARCH REPORT**

International application No. **PCT/CN2024/074207**

**A. CLASSIFICATION OF SUBJECT MATTER**

C07D401/12(2006.01)i; C07D401/14(2006.01)i; C07D403/14(2006.01)i; A61K31/4412(2006.01)i; A61K31/444(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, CNKI, ENTXTC, VEN, WEB OF SCIENCE, REGISTRY, CAPLUS: 齐鲁制药, PARP, 抑制剂, 吡啶, 嘧啶, 二嗪, inhibitor, pyridine, pyrimidine, diazine

**C. DOCUMENTS CONSIDERED TO BE RELEVANT**

| Category* | Citation of document, with indication, where appropriate, of the relevant passages | Relevant to claim No. |
|---|---|---|
| PX | WO 2023006013 A1 (SHANGHAI QILU PHARMACEUTICAL RESEARCH AND DEVELOPMENT CENTRE LTD.) 02 February 2023 (2023-02-02)<br>claims 32-38 | 1-35 |
| A | CN 115490671 A (SHUIMU WEILAI (BEIJING) TECHNOLOGY CO., LTD.) 20 December 2022 (2022-12-20)<br>claims 1-10 | 1-35 |
| A | WO 2021087018 A1 (RIBON THERAPEUTICS INC.) 06 May 2021 (2021-05-06)<br>claims 1-40, and description, page 37 | 1-35 |
| A | CN 115477640 A (YOULI BIOMEDICAL (SHANGHAI) CO., LTD.) 16 December 2022 (2022-12-16)<br>claims 1-10 | 1-35 |
| A | US 2022162196 A1 (RIBON THERAPEUTICS INC.) 26 May 2022 (2022-05-26)<br>claims 1-24 | 1-35 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 May 2024** | **07 May 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2024/074207**

| Patent document cited in search report | Publication date (day/month/year) | Patent family member(s) | Publication date (day/month/year) |
|---|---|---|---|
| WO 2023006013 A1 | 02 February 2023 | CA 3226796 A1 | 02 February 2023 |
| | | TW 202321224 A | 01 June 2023 |
| | | AU 2022316995 A1 | 14 March 2024 |
| CN 115490671 A | 20 December 2022 | None | |
| WO 2021087018 A1 | 06 May 2021 | None | |
| CN 115477640 A | 16 December 2022 | None | |
| US 2022162196 A1 | 26 May 2022 | WO 2020223229 A1 | 05 November 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310072130
- CN 202310072098


### Non-patent literature cited in the description


- **COHEN, P. CHANG**. Insights into the biogenesis, function, and regulation of ADP-ribosylation. *Nat Chem Biol*, 2018, vol. 14, 236-243
- **S.VYAS et al.** Family-wide analysis of poly (ADP-ribose) polymerase activity. *Nat Commun*, 2014, vol. 5, 4426
- **A. OHMOTO** ; **S. YACHIDA**. Current status of poly(ADP-ribose) polymerase inhibitors and future directions. *Onco Targets Ther*, 2017, vol. 10, 5195-5208
- **E. L. GOODE et al.** A genome-wide association study identifies susceptibility loci for ovarian cancer at 2q31 and 8q24. *Nat Genet*, 2010, vol. 42, 874-879
- **C.BIGETSBOLL et al.** TCDD-Inducible poly-ADP-ribose (TIPARP/PARP7) mono-ADP-ribosylates and co-activates liver X receptors. *Biochem J*, 2016, vol. 473, 899-910
- Guidelines for Hygroscopicity Test of Drugs. Chinese Pharmacopoeia. 2020